# EUROPÄISCHE PATENTSCHRIFT

(11) **EP 3 854 437 B1**
(45) Veröffentlichungstag und Bekanntmachung des Hinweises auf die Patenterteilung: **19.01.2022**
(21) Anmeldenummer: 20153853.5
(22) Anmeldetag: 27.01.2020
(51) Int. Cl.: A61M 25/00, A61M 25/01

(54) **VORRICHTUNG ZUR TEMPORÄREN, LOKALEN APPLIKATION VON FLUIDEN**
DEVICE FOR TEMPORARY LOCAL APPLICATION OF FLUIDS
DISPOSITIF D'APPLICATION TEMPORAIRE ET LOCALE DES FLUIDES

(43) Veröffentlichungstag der Anmeldung: 28.07.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- EP-A1- 2 656 869
- US-A- 4 306 563
- US-A- 4 968 306
- US-A1- 2006 229 573
- US-A1- 2013 274 711
- US-A1- 2014 094 773

## Beschreibung

Die Erfindung betrifft eine Vorrichtung zur temporären, lokalen Applikation von medizinischen Fluiden, insbesondere von pharmazeutischen Fluiden. Die Erfindung betrifft auch ein Verfahren zum Betreiben einer solchen Vorrichtung.

Gegenstand der Erfindung ist insbesondere eine medizinische Vorrichtung zur temporären, lokalen Applikation von pharmazeutischen Fluiden oder anderen medizinischen Fluiden über einen Zeitraum von Stunden bis zu mehreren Tagen. Die erfindungsgemäße Vorrichtung kann je nach der jeweiligen geometrischen Anforderung beziehungsweise je nach anatomischer Situation des Implantationsorts hinsichtlich ihrer Länge durch einfaches mechanisches Kürzen angepasst werden, ohne dass ein Funktionsverlust eintritt. Weiterhin wird eine Vorrichtung zum kontinuierlichen Austragen von medizinischen Fluiden vorgeschlagen, die mit der Vorrichtung zur lokalen Applikation von medizinischen Fluiden vorteilhaft kombiniert werden kann, so dass lokal über einen Zeitraum von Stunden bis Tagen pharmazeutische Fluide oder andere medizinische Fluide kontinuierlich lokal appliziert werden können.

Die lokale Applikation von pharmazeutischen Wirkstoffen wie Antibiotika ist schon seit Jahrzehnten bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden. Das Einleiten von Fluiden in Hohlräume zum Zweck der Spülung und Desinfektion kann aber auch zur Desinfektion und Reinigung von medizinischen Implantaten und Geräten mit Hohlräumen nützlich sein, deren Hohlräume ansonsten nur schwer zu erreichen wären.

Zur medizinischen Behandlung von Infektionen in schwer erreichbaren Hohlräumen und Kavitäten, wie Knochenhöhlen, sind resorbierbare und nichtresorbierbare Wirkstoffträger bekannt.

Exemplarisch für nichtresorbierbare Wirkstoffträger sind die seit 1977 unter dem Markennamen Septopal^{®} bekannten Kugelketten. Diese bestehen aus Polymethylmethacrylat-Kugeln, die das Breitbandantibiotikum Gentamicinsulfat enthalten, wobei diese Kugeln auf Stahlzwirn kettenförmig angeordnet sind (K. Klemm: Gentamcin-PMMA-beads in treating bone and soft tissue infections. Zentralbl. Chir. 104(14) (1979) 934-942.; K. Klemm: Antibiotic bead chains. Clin. Orthop. 295 (1993) 63-76.). Dieser kettenförmige Wirkstoffträger (Septopal^{®}) hat sich seit Jahrzehnten bei der lokalen antibiotischen Behandlung der Osteomyelitis bewährt. Vorteilhaft ist dabei, dass das Gentamicinsulfat in größeren Mengen über einen Zeitraum von mehreren Tagen aus dem Wirkstoffträger freigesetzt wird. Vorteilhaft ist es weiterhin, dass der kettenförmige Wirkstoffträger vom medizinischen Anwender problemlos durch einfaches Abschneiden des Stahlzwirns mit überzähligen Kugeln an die anatomische Situation am Implantationsort angepasst werden kann. Nachteilig ist, dass der Wirkstoffträger ausschließlich Gentamicinsulfat enthält und dass der medizinische Anwender den Wirkstoffträger nicht mit weiteren Antibiotika, entsprechend der Empfindlichkeit der mikrobiellen Keime, modifizieren kann. Zudem kann die Abgabe des pharmazeutischen Wirkstoffs nicht mehr ohne Austausch der Kugelkette an den Verlauf der Behandlung angepasst werden, wenn die Kugelkette erst einmal implantiert ist. Dadurch ist insbesondere die erfolgreiche lokale Behandlung von Infektionen mit Problemkeimen, wie MRSA und VRSA, nur bedingt oder nicht möglich. Die Entfernung der Kugelketten nach erfolgter Wirkstofffreisetzung ist durch Verwachsung mit Bindegewebe für den Patienten mit einer erheblichen Belastung verbunden.

Exemplarisch für resorbierbare beziehungsweise biodegradierbare Wirkstoffträger sind Vliese und Schwämme aus Kollagen oder Gelatine. Exemplarisch seien dafür die Druckschriften DE 34 29 038 A1, DE 28 43 963 C2, DE 32 03 957 C2 und DE 33 34 595 A1 genannt. Diese enthalten Gentamicinsulfat oder Gemische aus Gentamicinsulfat und einem in Wasser gering löslichen Gentamicinsalz. Weiterhin gibt es eine Vielzahl von resorbierbaren beziehungsweise biodegradierbaren Wirkstoffträgern auf Basis von Tricalciumphosphat, Hydroxylapatit, Gips und deren Mischungen sowie auch Kompositmaterialien aus diesen Salzen und organischen Bindemitteln. Eine Übersicht wurde von Kühn et al. publiziert (K.-D. Kühn, N. Renz, A. Trampuz: Lokale Antibiotika-Therapie. Der Unfallchirurg. 120 (2017) 561-572).

Nachteilig an den aufgeführten nicht-resorbierbaren und auch den resorbierbaren beziehungsweise biodegradierbaren Wirkstoffträgern ist, dass der antimikrobielle Wirkstoff durch die gewählte Zusammensetzung festgelegt ist und dass nach der Implantation des Wirkstoffträgers der Wirkstoff nicht mehr ausgetauscht oder mit anderen Wirkstoffen ergänzt werden kann. Weiterhin unterliegt die Wirkstofffreisetzung bei allen bisherigen lokalen Wirkstofffreisetzungssystemen auf dem Prinzip der Diffusion, so dass hohe Wirkstoffmengen nur in den ersten Stunden oder maximal ersten Tagen freigesetzt werden. Eine Ausnahme bildet die Verwendung von in Wasser gering löslichen Wirkstoffsalzen, bei denen die Wirkstofffreisetzung vom Löslichkeitsgleichgewicht der Wirkstoffsalze abhängt.

Wünschenswert ist daher ein Wirkstoffträger, der eine lokale Applikation beliebiger pharmazeutischer Wirkstoffe zulässt und wobei der pharmazeutische Wirkstoff jederzeit gegen andere fluide pharmazeutische Wirkstoffe ausgetauscht werden kann. Außerdem ist es wünschenswert, dass die Wirkstoffkonzentration, die unmittelbar am Implantationsort erreicht wird, direkt von außen eingestellt werden kann.

Aus der EP 2 656 869 A1 ist ein Katheter zur Behandlung von Veneninsuffizienz bekannt, der an seiner Vorderseite offen ist und bei dem an einem distalen Ende mehrere Löcher angeordnet sind, die mit einem äußeren Rohr teilweise abgedeckt werden können. Die US 2013/0274711 A1 offenbart einen Katheter mit radialen Öffnungen, bei dem die Anzahl von freiliegenden Öffnungen durch Verdrehen mehrerer ineinander liegender Rohre gegeneinander eingestellt werden kann. Aus der US 4 968 306 und der US 2006/0229573 A1 sind Katheter bekannt, bei denen seitliche Öffnungen mit einem äußeren Rohr abdeckbar sind. Die US 2014/0094773 A1 beschreibt einen Katheter mit einem Nabenelement, das an ein Rohr des Katheters gekoppelt werden kann.

Die EP 1 932 560 B1 offenbart einen Katheter zum Applizieren einer medizinischen Flüssigkeit. Der Katheter weist einen Schlauch auf, der an seinem distalen Schlauchende eine Vielzahl von Öffnungen aufweist, durch die eine Flüssigkeit aus dem Inneren des Schlauchs appliziert werden kann. Weitere ähnliche Katheter sind aus der US 5 800 407 A1, der US 6 537 194 A1 und der US 5 425 723 A1 bekannt. Diese Katheter haben den Nachteil, dass sie eine festgelegte Länge haben, über die sie die medizinische Flüssigkeit abgeben können, und sind daher nur für bestimmte Anwendungen und für Behandlungssituationen mit bestimmten geometrischen Abmessungen einsetzbar. Die Katheter weisen also nur eine sehr geringe Variabilität zur Anpassung an die Behandlungssituation auf. Zudem ist die Abgabe der medizinischen Flüssigkeit nur über einen sich langsam abbauenden Druck einstellbar, wobei der Druck von der Elastizität der die Flüssigkeit beinhaltenden Wandungen des Katheters abhängt. Eine schnelle und kurzfristige Abgabe der medizinischen Flüssigkeit ist nicht möglich. Ferner kann bei längerem Einsatz des Katheters (über mehr als einen Tag) das den Katheter umgebende Gewebe in die Öffnungen einwachsen und so beim Herausziehen / Entfernen des Katheters erhebliche Probleme verursachen. Das umgebende Gewebe kann so durch das Entfernen des Katheters beschädigt werden und dadurch der Behandlungserfolg gemindert werden.

Die nicht vorveröffentlichte EP 19 198 038 beschreibt eine Vorrichtung zur temporären, lokalen Applikation von medizinischen Fluiden mit einem Schlauch, der an seiner distalen Seite gekürzt werden kann. Dies kann nachteilig sein, wenn der distale Verschluss nicht ohne weiteres in den Schlauch eingesetzt werden kann. Gleichzeitig soll die medizinische Flüssigkeit aber nicht außerhalb des zur Behandlung vorgesehenen Hohlraums ausfließen.

Der Erfindung liegt die Aufgabe zugrunde, die Nachteile des Stands der Technik zu überwinden. Insbesondere soll eine Vorrichtung zur lokalen Applikation von medizinischen Fluiden, insbesondere von pharmazeutischen Fluiden, wie zum Beispiel von antibiotischen Lösungen, bereitgestellt werden, mit der eine lokale und temporäre Abgabe des medizinischen Fluids in schwer zugänglichen Bereichen ermöglicht wird, wie zum Beispiel in Hohlräumen von nicht implantierten Implantaten oder anderen medizinischen Vorrichtungen. Die Vorrichtung soll dabei flexibel an unterschiedliche Anwendungsbereiche anzupassen sein. Eine mechanische Belastung der Wandungen der zu spülenden Hohlräume soll dabei möglichst vermieden werden. Bei der Verwendung zur Behandlung einer Infektion soll eine möglichst schonende Behandlung möglich sein, bei der das angrenzende entzündete Gewebe möglichst wenig gereizt wird und zwar sowohl bei einer temporären Abgabe des Fluids als auch beim Einsetzen und Entfernen des eingeführten Teils der Vorrichtung. Die Vorrichtung soll auch über längere Zeiträume an einem bestimmten Ort zur wiederholten Abgabe des Fluids geeignet sein, ohne dass die Vorrichtung hierfür entfernt werden muss. Die Vorrichtung soll kostengünstig zu fertigen sein und möglichst ein hygienisches, nur einmal verwendbares Wegwerfprodukt sein. Dabei soll zumindest der in den zu spülenden Hohlraum platzierbare Teil der Vorrichtung oder eben die gesamte Vorrichtung als Wegwerfprodukt kostengünstig und leicht entsorgbar sein. Gleichzeitig soll die medizinische Flüssigkeit aber nicht außerhalb des zur Behandlung vorgesehenen Hohlraums ausfließen.

Die Aufgabe der Erfindung besteht somit auch darin, eine einfache, kostengünstige Vorrichtung zur lokalen Applikation von medizinischen Fluiden zu entwickeln. Die Vorrichtung soll eine lokale Applikation insbesondere von pharmazeutischen Fluiden mit beliebiger Zusammensetzung, zum Beispiel antibiotischen Lösungen, ermöglichen. Ein Teil der Vorrichtung befindet sich bei einer medizinischen Anwendung nach einer Implantation im Patienten und ein zweiter Teil der Vorrichtung außerhalb des Patienten. Die medizinischen Fluide sollen in dem außerhalb des im Patienten befindlichen Teils der Vorrichtung eingebracht werden können und zum Implantationsort durch die Vorrichtung geleitet und dort freigesetzt werden können. Die Vorrichtung soll plastisch verformbar sein, um den anatomischen Gegebenheiten am Implantationsort oder der geometrischen Form der Hohlform folgen zu können. Nach der erfolgten Formgebung durch den medizinischen Anwender soll die Gestalt der Vorrichtung sich nicht verändern können außer durch manuelle Verformung durch den medizinischen Anwender.

Die Freisetzung von pharmazeutischen Fluiden soll aus längs an der Vorrichtung angeordneten Öffnungen erfolgen. Die Öffnungen sollen reversibel verschließbar sein, um einen Rückfluss von kontaminiertem Fluid in das Innere der Vorrichtung zu verhindern beziehungsweise um ein Einwachsen von Bindegewebe beziehungsweise eine Verstopfung der Öffnungen durch koaguliertes Blut zu verhindern. Weiterhin soll die Vorrichtung so beschaffen sein, dass der gegebenenfalls im Patienten befindliche Teil der Vorrichtung durch Kürzen der Länge an die jeweilige anatomische Situation des Patienten angepasst werden kann, ohne dass die Funktion der Vorrichtung beeinträchtigt wird. Das Kürzen soll so erfolgen, dass an der nicht im Patienten befindlichen proximalen Seite der Vorrichtung gekürzt werden kann.

Die Vorrichtung darf sich außerdem nicht bei der Applikation des medizinischen Fluids beziehungsweise des pharmazeutischen Fluids hinsichtlich der Gestalt und auch hinsichtlich des Durchmessers signifikant verändern. Durch eine signifikante Querdehnung könnte es ansonsten zu Schmerzen am entzündeten beziehungsweise infizierten Gewebe beim Patienten kommen. Weiterhin soll eine einfache kostengünstige Vorrichtung entwickelt werden, die einen kontinuierlichen Austrag von medizinischen Fluiden, insbesondere von pharmazeutischen Fluiden, über einen Zeitraum von Stunden bis Tagen ermöglicht, ohne dass Elektromotoren, Batterien oder Akkumulatoren erforderlich sind.

Die Aufgaben der Erfindung werden gelöst durch eine Vorrichtung zur lokalen Applikation eines medizinischen Fluids aufweisend
einen Schlauch, der flexibel verformbar ist und der eine Schlauchwand aufweist,
wobei der Schlauch mehrere Öffnungen in der Schlauchwand aufweist, wobei die mehreren Öffnungen eine innere Leitung des Schlauchs mit der Umgebung des Schlauchs verbinden und wobei der Schlauch an einem distalen Schlauchende des Schlauchs verschlossen ist, wobei ein proximales Schlauchende des Schlauchs derart mit einem Behälter für das medizinische Fluid flüssigkeitsdurchlässig verbunden ist oder verbindbar ist, dass das medizinische Fluid aus dem Behälter durch das proximale Schlauchende des Schlauchs in die innere Leitung des Schlauchs drückbar ist und durch die mehreren Öffnungen in die Umgebung des Schlauchs herausdrückbar ist,
wobei die Vorrichtung eine äußere Hülse zum fluiddichten Verschließen eines Teils der mehreren Öffnungen aufweist, wobei die äußere Hülse axial verschiebbar um den Schlauch herum angeordnet ist und wobei die äußere Hülse kürzer ist als der Schlauch, so dass die nicht zum verschlossenen Teil der mehreren Öffnungen gehörenden distalen Öffnungen frei liegen, wobei
die Schlauchwand eine äußere Wandung aus einem ersten Material aufweist, die radial außen liegend angeordnet ist, und die Schlauchwand eine innere Wandung aus einem zweiten Material aufweist, die radial innen liegend angeordnet ist und die die innere Leitung des Schlauchs begrenzt, wobei
die äußere Wandung und die innere Wandung miteinander fest verbunden sind, bevorzugt vollflächig miteinander verbunden sind.

Bevorzugt ist der verschlossene Teil der mehreren Öffnungen ein proximaler Teil der mehreren Öffnungen. Der verschlossene Teil der mehreren Öffnungen liegt also bevorzugt auf der proximalen Seite des Schlauchs.

Mit der Vorrichtung können auch medizinische Instrumente und nicht implantierte Implantate abgespült oder ausgespült werden, insbesondere medizinischen Instrumente und Implantate mit Hohlräumen, in die der Schlauch eingeführt werden kann. Die Vorrichtung kann aber auch zum freien Verteilen des medizinischen Fluids verwendet werden. Besonders geeignet ist jedoch eine medizinische Anwendung der erfindungsgemäßen Vorrichtung, bei der der Schlauch in eine Kavität eines menschlichen Körpers eingeführt wird und das Fluid zur Behandlung des angrenzenden Gewebes eingesetzt wird.

Die erfindungsgemäße Vorrichtung ist bevorzugt eine medizinische Vorrichtung.

Vorzugsweise ist die äußere Hülse ein äußerer Schlauch oder ein äußeres Rohr zum fluiddichten Verschließen eines proximalen Teils der mehreren Öffnungen.

In dem distalen Schlauchende des Schlauchs, insbesondere in einem Verschlusselement, mit dem der Schlauch an dem distalen Schlauchende des Schlauchs flüssigkeitsdicht verschlossen ist, kann zumindest eine distale Öffnung angeordnet sein. Auch durch diese zumindest eine distale Öffnung kann das medizinische Fluid appliziert werden.

Bevorzugt ist der Schlauch plastisch verformbar.

Es kann vorgesehen sein, dass die äußere Hülse einen Innendurchmesser hat der gleich oder größer dem Außendurchmesser des Schlauchs ist. Bevorzugt hat die Hülse eine zylindrische Innenseite.

Gemäß einer bevorzugten Weiterbildung der vorliegenden Erfindung kann vorgesehen sein, dass in der proximalen Seite des Schlauchs ein Rückschlagventil angeordnet ist.

Es kann ferner vorgesehen sein, dass im Bereich des proximalen Schlauchendes des Schlauchs oder in der Verbindung zu dem Behälter für das medizinische Fluid ein Ventilelement, insbesondere ein Rückschlagventil, angeordnet ist, das eine Strömung des medizinischen Fluids in Richtung des Behälters verhindert und eine Strömung des medizinischen Fluids aus dem Behälter in Richtung des distalen Teilstücks verhindert.

Hierdurch wird sichergestellt, dass kein kontaminiertes medizinisches Fluid aus der inneren Leitung in den Behälter für das medizinische Fluid vordringen kann.

Die Richtungsbezeichnungen distal und proximal beziehen sich vorliegend auf die in der Anwendung vorgesehene Flussrichtung des medizinischen Fluids. Das medizinische Fluid fließt dabei von einem proximalen Schlauchende des Schlauchs in Richtung zum distalen Schlauchende des Schlauchs und dort aus den mehreren Öffnungen.

Die Schlauchwand kann mantelförmig geformt sein.

Die Richtungsangabe axial bezieht sich auf die Symmetrieachse des Schlauchs, wenn dieser gerade ausgerichtet ist.

Bevorzugt ist der Schlauch bis auf die mehreren Öffnungen zylindrisch geformt. Die Schlauchwand begrenzt den Schlauch dann besonders bevorzugt an seiner Zylindermantelfläche. Bei geraden Schläuchen mit einer zylindrischen Geometrie ist die Mantelfläche die Wandung senkrecht zur Zylinderachse des zylindrischen Schlauchs. Die Öffnungen befinden sich also in der Mantelfläche.

Als medizinisches Fluid wird bevorzugt ein pharmazeutisches Fluid verwendet. Ein pharmazeutisches Fluid enthält wenigstens einen pharmazeutischen Wirkstoff. Besonders bevorzugt werden Lösungen enthaltend wenigstens ein Antibiotikum, wenigstens ein Zytostatikum, wenigstens ein Chemotherapeutikum und/oder wenigstens ein Antimykotikum pharmazeutische Fluide beziehungsweise als medizinische Fluide. Alternative medizinische Fluide können desinfizierende Bestandteile enthalten. Unter dem Begriff "pharmazeutisches Fluid" werden demzufolge wässrige und auch nichtwässrige Lösungen und Suspensionen von pharmazeutischen Wirkstoffen verstanden. Weiterhin werden unter dem Begriff "pharmazeutisches Fluid" auch Gemische und Lösungen von Gasen in Wasser, Wasser enthaltenden Flüssigkeiten und nichtwässrigen Flüssigkeiten verstanden. Der Begriff "pharmazeutisches Fluid" umfasst bevorzugt auch Gase und Gasgemische.

Es kann auch vorgesehen sein, dass zumindest eine der mehreren Öffnungen im Bereich des distalen Schlauchendes des Schlauchs angeordnet ist, bevorzugt innerhalb von 5 mm des distalen Schlauchendes des Schlauchs angeordnet ist, besonders bevorzugt innerhalb von 3 mm des distalen Schlauchendes des Schlauchs angeordnet ist.

Bevorzugt sind in der Schlauchwandung zumindest drei Öffnungen als die mehreren Öffnungen angeordnet.

Auch kann vorgesehen sein, dass die mehreren Öffnungen alle, paarweise oder gruppenweise in axialer Richtung des äußeren Schlauchs zueinander beabstandet sind.

Hierdurch kann das medizinische Fluid an verschiedenen axial voneinander beabstandeten Stellen austreten. Zudem kann der Schlauch auf der proximalen Seite in der Länge gekürzt werden, wobei gleichzeitig immer noch wenigstens eine Öffnung der mehreren Öffnungen in einem distalen Teilstück des Schlauchs vorhanden ist.

Die äußere Hülse hat einen Innendurchmesser gleich oder größer dem Außendurchmesser des Schlauchs.

Die äußere Hülse hat eine kürzere Länge als der Schlauch.

Die äußere Hülse hat bevorzugt eine Länge von 10 bis 15 cm. Diese Länge reicht bei durchschnittlichen anatomischen Verhältnissen aus, um von einem Röhrenknochen bis über die überdeckende Weichgewebsschicht die unter der äußeren Hülse liegenden Öffnungen des Schlauchs so zu verdecken, dass ein unerwünschter Austritt des pharmazeutischen Fluids außerhalb des zu behandelnden Röhrenknochens und der Hautoberfläche verhindert wird.

Es ist vorgesehen, dass die Schlauchwand eine äußere Wandung aus einem ersten Material aufweist, die radial außen liegend angeordnet ist, und die Schlauchwand eine innere Wandung aus einem zweiten Material aufweist, die radial innen liegend angeordnet ist und die die innere Leitung des Schlauchs begrenzt.

Mit den unterschiedlichen Materialien kann der Schlauch als ein Verbundwerkstoff aufgebaut werden, wobei die äußere Wandung und die innere Wandung unterschiedlich auf physikalische oder chemische Parameter, wie Druck oder Temperatur, reagieren können, so dass bestimmte gewünschte Veränderungen von Eigenschaften, wie Form oder Festigkeit des Schlauchs als Reaktion auf eine Veränderung der physikalischen oder chemischen Parameter eingestellt werden können. Hierdurch können beispielsweise die mehreren Öffnungen, die durch die Schlauchwand gehen, bei geeigneter Auswahl des ersten und des zweiten Materials mit Hilfe eines veränderbaren hydrostatischen Drucks geöffnet und geschlossen werden.

Das erste Material und das zweite Material unterscheiden sich bevorzugt hinsichtlich zumindest einer Materialeigenschaft voneinander. Besonders bevorzugt unterscheiden sich das erste Material und das zweite Material hinsichtlich der Elastizität und/oder Härte voneinander.

Gemäß einer bevorzugten Weiterbildung der vorliegenden Erfindung kann das zweite Material ein gummielastisches Material sein, während das erste Material formstabiler als das zweite Material ist. Beispielsweise kann die innere Wandung eine Beschichtung mit einem gummielastischen Material auf der Innenseite der äußeren Wandung sein.

Die äußere Wandung kann die innere Wandung mantelförmig umgeben.

Bei erfindungsgemäßen Vorrichtungen mit äußerer Wandung und innerer Wandung ist auch vorgesehen, dass die äußere Wandung und die innere Wandung miteinander fest verbunden sind, bevorzugt vollflächig miteinander verbunden sind.

Hiermit sind die äußere Wandung und die innere Wandung gegeneinander fixiert. Dadurch ist es möglich, dass die mehreren Öffnungen in der inneren Wandung sich aufgrund einer elastischen Entspannung ohne einen Druck des medizinischen Fluids in der inneren Leitung verschließen können, während die äußere Wandung den Druck aufnehmen kann, der zum Öffnen der mehreren Öffnungen in der inneren Wandung notwendig ist.

Des Weiteren kann vorgesehen sein, dass die mehreren Öffnungen in der äußeren Wandung der Schlauchwand unabhängig vom Druck des medizinischen Fluids offen sind, während die mehreren Öffnungen in der inneren Wandung der Schlauchwand ohne Druckbeaufschlagung durch das medizinische Fluid verschlossen sind und durch einen Druck auf das medizinische Fluid flüssigkeitsdurchlässig zu öffnen sind.

Hierdurch verschließen sich die mehreren Öffnungen in der Schlauchwand, wenn keine medizinische Flüssigkeit in die innere Leitung gedrückt wird. Bei einem alternierenden Betrieb kann so verhindert werden, dass Gewebe durch die mehreren Öffnungen in die innere Leitung wächst und so die Vorrichtung in der Kavität verwächst.

Bevorzugt kann auch vorgesehen sein, dass die äußere Wandung der Schlauchwand einen über die innere Wandung der Schlauchwand vermittelten Druck des medizinischen Fluids in der inneren Leitung aufnimmt, ohne sich dabei radial um mehr als 5% auszudehnen, bevorzugt ohne sich dabei radial um mehr als 1% auszudehnen.

Der Druck der medizinischen Flüssigkeit kann sich dabei unter normalen Bedingungen bei normalen Anwendungen der erfindungsgemäßen Vorrichtung nicht über 500 kPa erhöhen. Es kann also vorgesehen sein, dass die äußere Wandung der Schlauchwand einen über die innere Wandung der Schlauchwand vermittelten hydrostatischen Druck von maximal 500 kPa in der inneren Leitung aufnimmt, ohne sich dabei radial um mehr als 5% auszudehnen, bevorzugt ohne sich dabei radial um mehr als 1% auszudehnen.

Hierdurch wird sichergestellt, dass sich der Schlauch nicht zu stark ausdehnt, wenn das medizinische Fluid durch den Schlauch gedrückt wird. Hiermit wird eine Irritation des umgebenden Gewebes verhindert oder eine mechanische Belastung der umgebenden Strukturen verhindert.

Auch kann vorgesehen sein, dass das erste Material eine größere Shore A-Härte hat als das zweite Material, wobei bevorzugt das erste Material eine Shore A-Härte von mehr als 60 und das zweite Material eine Shore A-Härte von weniger als 60 hat.

Die Shore-Härte wird dazu nach DIN ISO 7619-1 (2012-02) [2] bestimmt. Durch Materialien mit diesen Härteunterschieden wird sichergestellt, dass die innere Wandung die mehreren Öffnungen in der äußeren Wandung verschließen kann.

Ferner kann vorgesehen sein, dass die mehreren Öffnungen in der inneren Wandung bei Druckbeaufschlagung mit einem hydrostatischen Druck von 500 kPa durch das medizinische Fluid einen um den Faktor zwei oder mehr größeren freien Querschnitt aufweisen als ohne Druckbeaufschlagung.

Hiermit wird sichergestellt, dass die mehreren Öffnungen in der inneren Wandung durch den Druck des medizinischen Fluids zu öffnen sind.

Es kann vorgesehen sein, dass die mehreren Öffnungen in der inneren Wandung oder der äußeren Wandung schlitzförmig geformt sind.

Durch diese beiden Maßnahmen wird erreicht, dass sich die schlitzförmigen Öffnungen durch einen auf das medizinische Fluid wirkenden Druck öffnen lassen und sich bei Reduzieren des Drucks auf das medizinische Fluid wieder verschließen. Dadurch kann ein Einwachsen von Gewebe in die mehreren Öffnungen bei alternierendem Betrieb vermieden werden. Auch kann so eine Verunreinigung des medizinischen Fluids in der inneren Leitung verhindert werden. Bevorzugt sind die mehreren Öffnungen in der inneren Wandung schlitzförmig geformt. Das hat den Vorteil, dass deren Verformung keine Verformung der Außenflächen des Schlauchs verursacht.

Des Weiteren kann vorgesehen sein, dass die mehreren Öffnungen im Schlauch in der äußeren Wandung einen Durchmesser von maximal 500 µm, bevorzugt von maximal 250 µm und besonders bevorzugt von maximal 100 µm haben.

Der Durchmesser bezieht sich dabei auf den mittleren Durchmesser des Öffnungsquerschnitts der mehreren Öffnungen. Wenn die innere Wandung dazu ausgelegt ist, die mehreren Öffnungen im entspannten Zustand, also bei fehlender Druckbeaufschlagung, zu verschließen, ist der Durchmesser der inneren Wandung zumindest im geschlossenen Zustand natürlich kleiner. Im geöffneten Zustand der mehreren Öffnungen in der inneren Wandung ist der Durchmesser der mehreren Öffnungen in der inneren Wandung maximal so groß wie der Durchmesser der mehreren Öffnungen in der äußeren Wandung.

Bei Öffnungen mit solchen maximalen Durchmessern ist sichergestellt, dass die Durchflussraten des medizinischen Fluids nicht zu hoch werden und andererseits der freie Leitungsquerschnitt der inneren Leitung dazu ausreicht, auch die Öffnungen nahe dem distalen Schlauchende des Schlauchs nur zur Abgabe des medizinischen Fluids nutzen zu können.

Bevorzugte Ausführungsformen der vorliegenden Erfindung können sich auch dadurch auszeichnen, dass der Schlauch aus einem koaxialen Koextrudat gebildet ist, wobei die innere Wandung aus einem gummielastischen Polymer, insbesondere Polyurethan oder einem schwach vernetzten Polymer, besteht und die äußere Wandung aus einem nicht gummielastischen thermoplastischen Polymer oder aus einem stark vernetzten Polymer besteht, insbesondere aus Polyamid besteht.

Hierdurch wird erreicht, dass die Öffnungen durch die innere Wandung verschlossen sind, wenn kein hydrostatischer Druck auf die innere Wandung ausgeübt wird, und sich die Öffnungen in der inneren Wandung öffnen, wenn der von dem medizinischen Fluid ausgeübte Druck ansteigt. Dadurch kann ein Einwachsen von Gewebe und eine Verunreinigung der inneren Leitung durch die Öffnungen verhindert werden.

Besonders bevorzugt sind koextrudierte Schläuche bei denen die inneren Wandung aus einem gummielastischen Polyurethan besteht und die äußere Wandung aus thermoplastischem, nicht gummielastischem Polyamid gebildet ist. Die mehreren Öffnungen gehen durch die innere Wandung und die äußere Wandung. Bei Druckbeaufschlagung mit einem medizinischen Fluid werden die Öffnungen in der inneren Wandung durch elastisches Zurückweichen des elastischen Polyurethans freigeben und das medizinische Fluid kann durch die Öffnungen des starren Polyamids aus der äußeren Wandung austreten. Nach Beendigung des Fluidaustrags verschließen sich die Öffnungen in der inneren Wandung des Schlauchs wieder und Körperflüssigkeiten, wie zum Beispiel Blut oder Wundexsudat, können nicht in die innere Leitung des Schlauchs eindringen. Währen des Fluidaustrags verhindert die starre, nichtelastische äußere Wandung eine radiale Dehnung des Schlauchs. Dadurch werden keine Kräfte auf das zu behandelnde Gewebe ausgeübt und Schmerzen infolge einer mechanischen Einwirkung verhindert, beziehungsweise keine Kräfte auf die Wandungen des gespülten Implantats oder Hohlraums übertragen.

Bei erfindungsgemäßen Vorrichtungen mit einer inneren Wandung und einer äußeren Wandung kann vorgesehen sein, dass die mehreren Öffnungen in der inneren Wandung abhängig von einer auf das zweite Material wirkenden physikalischen Größe, insbesondere abhängig von einem von dem medizinischen Fluid auf das zweite Material wirkenden Druck, reversibel fluiddicht verschließbar sind.

Alternativ kann vorgesehen sein, dass die mehreren Öffnungen in der äußeren Wandung abhängig von einer auf das erste Material wirkenden physikalischen Größe, insbesondere abhängig von einem von dem medizinischen Fluid auf das erste Material wirkenden Druck, reversibel fluiddicht verschließbar sind.

Im zweiten Fall kann der Druck in den offenen Teilen der mehreren Öffnungen in der inneren Wandung auf das erste Material der äußeren Wandung wirken. Bevorzugt sind das erste Material und das zweite Material derart gewählt, dass die mehreren Öffnungen in der inneren Wandung abhängig von einer auf das zweite Material wirkenden physikalischen Größe, insbesondere abhängig von einem von dem medizinischen Fluid auf das zweite Material wirkenden Druck, reversibel fluiddicht verschließbar sind oder die mehreren Öffnungen in der äußeren Wandung abhängig von einer auf das erste Material wirkenden physikalischen Größe, insbesondere abhängig von einem von dem medizinischen Fluid auf das erste Material wirkenden Druck, reversibel fluiddicht verschließbar sind.

Durch diese Maßnahmen können die mehreren Öffnungen reversibel geöffnet und verschlossen werden. Neben dem Druck kann beispielsweise eine elektrische oder mechanische Spannung, ein Magnetfeld oder eine Temperatur (beispielsweise durch den Einsatz von Shape-Memory-Alloys) zum Öffnen und Schließen der mehreren Öffnungen verwendet werden.

Des Weiteren kann vorgesehen sein, dass die mehreren Öffnungen durch eine elastische Deformation des zweiten Materials reversibel zu öffnen sind, während die mehreren Öffnungen im ersten Material offen bleiben, wobei vorzugsweise das erste Material derart formfest ist, dass die äußere Wandung zumindest einen Teil der durch die elastische Verformung des zweiten Materials verursachten Kräfte aufnimmt und so einer radialen Verformung des Schlauchs entgegenwirkt.

Hierdurch wird erreicht, dass der Schlauch sich nicht oder nur sehr gering radial verformt.

Alternativ kann auch vorgesehen sein, dass die mehreren Öffnungen durch eine elastische Deformation des ersten Materials reversibel zu öffnen sind, während die mehreren Öffnungen im zweiten Material offen bleiben, wobei vorzugsweise das zweite Material derart formfest ist, dass die innere Wandung zumindest einen Teil der durch die elastische Verformung des ersten Materials verursachten Kräfte aufnimmt und so einer radialen Verformung des Schlauchs entgegenwirkt.

Mit der vorliegenden Erfindung wird auch vorgeschlagen, dass die Vorrichtung ein Verschlusselement aufweist, mit dem der Schlauch an dem distalen Schlauchende des Schlauchs flüssigkeitsdicht verschlossen ist.

Hiermit wird der Aufbau der Vorrichtung vereinfacht. Das Verschlusselement kann einen mechanischen Verschluss des Schlauchs bewirken und durch eine Spannung des Schlauchs abdichten.

Es kann auch vorgesehen sein, dass die innere Leitung des Schlauchs an einer proximalen Öffnung im proximalen Schlauchende des Schlauchs beginnt und an einer distalen Öffnung im distalen Schlauchende des Schlauchs endet, wobei vorzugsweise die distale Öffnung des Schlauchs durch das Verschlusselement verschlossen ist.

Die innere Leitung kann so die beiden offenen Enden, nämlich das distale Schlauchende und das proximale Schlauchende des Schlauchs, verbinden. Hierdurch kann das medizinische Fluid durch die innere Leitung des Schlauchs geleitet und durch die mehreren Öffnungen in der Schlauchwand appliziert werden.

Bevorzugt ist das Verschlusselement in das distale Schlauchende des Schlauchs eingeschraubt oder eingepresst, so dass es die innere Leitung über deren gesamten Querschnitt am distalen Schlauchende vollständig flüssigkeitsdicht und besonders bevorzugt auch gasdicht verschließt.

Des Weiteren kann vorgesehen sein, dass der Schlauch am distalen Schlauchende mit dem Verschlusselement gasdicht und/oder druckdicht verschlossen ist oder verschließbar ist.

Ferner kann vorgesehen sein, dass die Vorrichtung den Behälter für das medizinische Fluid aufweist, wobei bevorzugt der Behälter einen Hohlzylinder mit einem axial im Hohlzylinder verschiebbaren Kolben aufweist, der ein erstes Ende des Hohlzylinders verschließt, wobei der Hohlzylinder an einem dem ersten Ende gegenüberliegenden Ende eine Austragsöffnung aufweist, die mit dem proximalen Schlauchende des Schlauchs verbunden oder verbindbar ist, bevorzugt über ein manuell bedienbares Ventilelement zur Regulierung der Strömungsgeschwindigkeit des medizinischen Fluids mit dem proximalen Schlauchende des Schlauchs verbunden oder verbindbar ist.

Hierdurch muss kein separates Reservoir für die medizinische Flüssigkeit an der Vorrichtung angeschlossen werden. Bevorzugt ist der Kolben mit zumindest einer gespannten elastischen Feder antreibbar.

Auch kann vorgesehen sein, dass in dem Behälter ein medizinisches Fluid, insbesondere ein pharmazeutisches Fluid, enthalten ist.

Hierdurch ist die Vorrichtung unmittelbar zum Erzeugen eines Stroms des medizinischen Fluids aus den mehreren Öffnungen einsetzbar.

Gemäß einer bevorzugten Weiterbildung der vorliegenden Erfindung kann vorgesehen sein, dass die Vorrichtung eine Fördereinrichtung aufweist, mit der das medizinische Fluid aus dem verbundenen oder verbindbaren Behälter in den Schlauch, durch die innere Leitung des Schlauchs und durch die mehreren Öffnungen in die Umgebung des Schlauchs zu drücken ist.

Damit kann die Vorrichtung auch zum Antreiben des Stroms der medizinischen Flüssigkeit verwendet werden. Mit einer solchen Vorrichtung ist es möglich, über einen Zeitraum von Stunden bis zu mehreren Tagen pharmazeutische Fluide lokal zu applizieren, ohne dass aufwendige elektrisch betriebene Pumpsysteme notwendig sind. Bevorzugt kann mit der Fördereinrichtung das medizinische Fluid diskontinuierlich oder kontinuierlich gefördert werden.

Bei Vorrichtungen mit Fördereinrichtung kann bevorzugt vorgesehen sein, dass die Fördereinrichtung ein Energiespeicherelement, insbesondere zumindest eine gespannte Feder, aufweist, wobei die Fördereinrichtung mit Energie aus dem Energiespeicherelement antreibbar ist, wobei besonders bevorzugt mit dem Energiespeicherelement ein Kolben in einem Hohlzylinder als der Behälter in Richtung einer gegenüberliegenden Austragsöffnung anzutreiben ist.

Dadurch muss die Vorrichtung nicht an eine externe Energieversorgung zum Antreiben der Fördereinrichtung angeschlossen werden. Eine gespannte Feder enthält dabei genug Energie, um eine Menge von einigen Millilitern bis einigen Zentilitern des medizinischen Fluids mit der Vorrichtung auszupressen.

Ferner kann vorgesehen sein, dass die Vorrichtung einen Konnektor zum Anschließen des Schlauchs an den Behälter für das medizinische Fluid aufweist, wobei der Konnektor einen konischen oder zylindrischen Vorsprung aufweist, der in den Schlauch gesteckt oder geschraubt ist, so dass der Schlauch im Bereich des proximalen Schlauchendes des Schlauchs durch den konischen oder zylindrischen Vorsprung derart gespannt ist, dass der Schlauch am proximalen Schlauchende des Schlauchs fluiddicht mit dem Behälter verbunden ist und fest mit der äußeren Hülse verbunden ist.

Hiermit lässt sich das proximale Schlauchende des Schlauchs auch nach einem Kürzen eines proximalen Teils des Schlauchs zuverlässig abdichten.

Dabei kann vorgesehen sein, dass der konische oder zylindrische Vorsprung Stege an der Außenseite des konischen oder zylindrischen Vorsprungs aufweist oder der konische oder zylindrische Vorsprung ein Außengewinde aufweist, wobei das Außengewinde oder der konische oder zylindrische Vorsprung einen größeren Außendurchmesser aufweist als der Innendurchmesser des Schlauchs.

Es kann erfindungsgemäß vorgesehen sein, dass der Schlauch plastisch verformbar ist und einen Außendurchmesser kleiner oder gleich 7 mm aufweist, bevorzugt einen Außendurchmesser zwischen 2 mm und 4 mm.

Durch den geringen Außendurchmesser ist der Schlauch gut in Kavitäten von Implantaten und in Kavitäten im menschlichen Körper einführbar und dort zum Ausspülen der Kavitäten geeignet. Durch die plastische Verformbarkeit wird verhindert, dass eine elastische Kraft auf die Wandungen des zu spülenden Hohlraums wirkt und diesen mechanisch belastet.

Des Weiteren kann vorgesehen sein, dass zumindest am distalen Schlauchende des Schlauchs und/oder im Verschlusselement ein röntgenopakes Material im Schlauch enthalten ist, bevorzugt in einem distalen Teilstück des Schlauchs und, sofern vorhanden, im Verschlusselement ein röntgenopakes Material enthalten ist, besonders bevorzugt auf der gesamten Länge des Schlauchs und, sofern vorhanden, im Verschlusselement enthalten ist.

Hierdurch kann die Position und Lage des Schlauchs durch Röntgenverfahren sichtbar gemacht werden und der Schlauch unter Röntgenkontrolle positioniert werden und dadurch die Lage der Vorrichtung im Patienten durch Röntgenaufnahmen eindeutig bestimmt werden.

Das röntgenopake Material kann besonders bevorzugt ausgewählt sein aus Edelstahl, Titan, Titanlegierungen, Tantal, Tantallegierungen, Bariumsulfat, Bariumsulfat enthaltende Kunststoffe, Zirkoniumdioxid und Zirkoniumdioxid enthaltende Kunststoffe.

Ferner kann vorgesehen sein, dass zumindest ein Metalldraht, zumindest eine Metallspirale und/oder zumindest ein Metallnetz in der inneren Leitung des Schlauchs und/oder in der Schlauchwand des Schlauchs angeordnet ist oder sind, wobei der zumindest eine Metalldraht, die zumindest eine Metallspirale und/oder das zumindest ein Metallnetz bevorzugt entlang der gesamten Länge des Schlauchs angeordnet sind.

Der zumindest eine Metalldraht, die zumindest eine Metallspirale und das zumindest eine Metallnetz dienen der plastischen Verformbarkeit des Schlauchs. Dadurch kann die Form des Schlauchs verändert und damit an die jeweilige Situation angepasst werden, ohne dass die Umgebung der Vorrichtung mechanisch beansprucht wird, wobei die Form des Schlauchs von dem zumindest einen Metalldraht, der zumindest einen Metallspirale und/oder dem zumindest ein Metallnetz gehalten wird. So behält die Vorrichtung nach vorheriger Formung entsprechend den anatomischen Gegebenheiten ihre Form bei. Dadurch ist ein ortsgenaues Applizieren von pharmazeutischen Fluiden an genau vorherbestimmten Implantationsorten möglich. Zudem sind die metallischen Strukturen im Röntgenbild erkennbar.

Bevorzugt kann vorgesehen sein, dass in dem Verschlusselement ein röntgenopakes Material enthalten ist oder dass das Verschlusselement aus einem röntgenopaken Material besteht. Das röntgenopake Material kann besonders bevorzugt ausgewählt sein aus Edelstahl, Titan, Titanlegierungen, Tantal, Tantallegierungen, Bariumsulfat, Bariumsulfat enthaltende Kunststoffe, Zirkoniumdioxid und Zirkoniumdioxid enthaltende Kunststoffe.

Bevorzugt kann vorgesehen sein, dass die Summe der freien Querschnittsflächen aller der mehreren Öffnungen maximal so groß ist wie der freie Querschnitt der inneren Leitung.

Hierdurch wird sichergestellt, dass auch die Öffnungen der mehreren Öffnungen, die am distalen Schlauchende des Schlauchs angeordnet sind, von dem medizinischen Fluid durchströmt werden können. Damit wird sichergestellt, dass auch aus den am distalen Schlauchende angeordneten Öffnungen noch medizinisches Fluid austritt. Die Summe der freien Querschnittsflächen aller der mehreren Öffnungen bezieht sich den geöffneten Zustand der mehreren Öffnungen.

Mit der vorliegenden Erfindung wird auch vorgeschlagen, dass sich der Schlauch bei einem Innendruck von 500 kPa gegenüber Normaldruck sich radial um maximal 10 Prozent ausdehnt, bevorzugt um maximal 5 Prozent ausdehnt.

Hierdurch wird sichergestellt, dass sich der Schlauch nicht zu stark ausdehnt, wenn das medizinische Fluid durch den Schlauch gedrückt wird. Hiermit wird eine Irritation des umgebenden Gewebes verhindert oder eine mechanische Belastung der umgebenden Strukturen verhindert.

Bevorzugt kann auch vorgesehen sein, dass das Verschlusselement die folgenden Merkmale aufweist: einen rotationssymmetrischen ersten Körper mit Außengewinde oder mit am Umfang umlaufenden Stegen, wobei das Außengewinde oder die Stege einen größeren Außendurchmesser besitzt als der Innendurchmesser des Schlauchs, einen rotationssymmetrischen zweiten Körper mit einem Außendurchmesser kleiner oder gleich dem Außendurchmesser des Schlauchs, wobei die axiale Erstreckung des zweiten Körpers mindestens 5 mm ist, wobei der rotationssymmetrische erste Körper axial mit dem rotationssymmetrischen zweiten Körper verbunden ist.

Hierdurch kann der Schlauch mit dem Verschlusselement zuverlässig und flüssigkeitsdicht verschlossen werden.

Es kann auch vorgesehen sein, dass das Verschlusselement in das distale Schlauchende des Schlauchs eingeschraubt oder eingepresst ist und den freien Leitungsquerschnitt der inneren Leitung des Schlauchs am distalen Schlauchende vollständig flüssigkeitsdicht und druckdicht verschließt.

Hierdurch wird sichergestellt, dass in der inneren Leitung ein ausreichender Druck mit dem medizinischen Fluid aufgebaut werden kann, um die medizinische Flüssigkeit aus allen der mehreren Öffnungen drücken zu können. Bevorzugt verschließt das Verschlusselement das distale Schlauchende des Schlauchs auch gasdicht.

Die der vorliegenden Erfindung zugrundeliegenden Aufgaben werden auch gelöst durch ein Verfahren zum Anpassen der Schlauchlänge einer medizinischen Vorrichtung zur lokalen Applikation eines medizinischen Fluids, die Vorrichtung aufweisend einen Schlauch mit einer Schlauchwand, wobei der Schlauch mehrere Öffnungen in der Schlauchwand aufweist, wobei die mehreren Öffnungen eine innere Leitung des Schlauchs mit der Umgebung des Schlauchs verbinden, und die Vorrichtung aufweisend eine äußere Hülse zum fluiddichten Verschließen eines proximalen Teils der mehreren Öffnungen, wobei die äußere Hülse axial verschiebbar um den Schlauch herum angeordnet ist und wobei die äußere Hülse kürzer ist als der Schlauch, so dass die nicht zum proximalen Teil der mehreren Öffnungen gehörenden Öffnungen frei liegen, das Verfahren aufweisend die folgenden Schritte:
A) Verschieben der äußeren Hülse auf dem Schlauch bis eine gewünschte distale Länge des Schlauchs freiliegt;
B) Abschneiden des über die äußere Hülse überstehenden proximalen Teils des Schlauchs;
C) Fixieren der äußeren Hülse gegen den Schlauch; und
D) Verbinden des neuen proximalen Schlauchendes des Schlauchs mit einem Behälter für das medizinische Fluid oder mit einem Anschluss für einen Behälter für das medizinische Fluid, so dass der Behälter flüssigkeitsdurchlässig mit der inneren Leitung des Schlauchs verbunden ist oder verbindbar ist.

Die Schritte werden vorzugsweise chronologisch nacheinander durchgeführt.

Hiermit kann der Schlauch auf einfache Weise auf eine zur Anwendung passende Länge gebracht werden.

Bei erfindungsgemäßen Verfahren ist vorgesehen, dass bei dem Verfahren keine medizinische Behandlung eines menschlichen oder tierischen Körpers erfolgt und/oder das medizinische Fluid im Rahmen des Verfahrens nicht an einen menschlichen oder tierischen Körper abgegeben wird.

Hiermit wird klargestellt, dass es sich bei dem erfindungsgemäßen Verfahren nicht um ein Verfahren zur Behandlung des menschlichen Körpers handelt.

Die der vorliegenden Erfindung zugrundeliegenden Aufgaben werden auch gelöst durch ein Verfahren zum Betreiben einer medizinischen Vorrichtung zur lokalen Applikation eines medizinischen Fluids umfassend ein erfindungsgemäßes Verfahren zum Anpassen der Schlauchlänge einer medizinischen Vorrichtung zur lokalen Applikation eines medizinischen Fluids, wobei die Schlauchwand des Schlauchs der Vorrichtung eine äußere Wandung aus einem ersten Material aufweist, die radial außen liegend angeordnet ist, und die Schlauchwand eine innere Wandung aus einem zweiten Material aufweist, die radial innen liegend angeordnet ist und die die innere Leitung des Schlauchs begrenzt, gekennzeichnet durch die folgenden Schritte:
Schritt E) Einleiten eines medizinischen Fluids in den Schlauch;
Schritt F) Ausüben eines Drucks auf das medizinische Fluid in dem Schlauch;
Schritt G) Öffnen der mehreren Öffnungen in der inneren Wandung oder in der äußeren Wandung des Schlauchs durch den auf die mehreren Öffnungen (4) wirkenden Druck des medizinischen Fluids; und
Schritt H) Austreiben von medizinischem Fluid durch die geöffneten mehreren Öffnungen.

Nach Schritt H) kann der Druck auf das medizinische Fluid reduziert werden und dadurch die mehreren Öffnungen verschlossen werden.

Des Weiteren kann vorgesehen sein, dass ein folgender optionaler Schritt I) erfolgt: Schritt I) Reduzieren des Drucks auf das medizinische Fluid in dem Schlauch nach Schritt H) und dadurch Schließen der mehreren Öffnungen in der inneren Wandung des Schlauchs oder Verringern des freien Querschnitts der mehreren Öffnungen in der inneren Wandung des Schlauchs.

Hierdurch kann das Verfahren alternierend eingesetzt werden, ohne dass das medizinische Fluid in der inneren Leitung durch rückströmende Fluide kontaminiert wird.

Es kann erfindungsgemäß vorgesehen sein, dass das Verfahren mit einer erfindungsgemäßen Vorrichtung durchgeführt wird.

Hierdurch ergeben sich für das Verfahren die zu den jeweiligen Ansprüchen genannten Vorteile.

Der Erfindung liegt die überraschende Erkenntnis zugrunde, dass der Schlauch problemlos gekürzt werden kann und so dessen Länge an die jeweilige Situation angepasst werden kann und zwar auch dann, wenn das distale Schlauchende des Schlauchs sich bereits in dem Hohlraum befindet, in dem das medizinische Fluid appliziert werden soll. Hierzu muss lediglich das proximale Schlauchende des Schlauchs nach dem Abschneiden mit einem vorhandenen Konnektor oder einem anderen Anschluss an den Behälter für das medizinische Fluid angeschlossen werden. Ein Austritt durch Öffnungen des Schlauchs, die nicht innerhalb des Hohlraums angeordnet sind, wird dabei dadurch verhindert, dass die äußere Hülse die außen liegenden Öffnungen der mehreren Öffnungen verschließt, die auf der proximalen Seite des Schlauchs angeordnet sind. Durch eine Weitung des Schlauchs auf der proximalen Seite durch Einstecken eines Konnektors oder eines anderen weitenden Anschlusses, kann die Außenwand des äußeren Schlauchs gegen die Innenwand der äußeren Hülse gepresst werden und dadurch eine Fixierung und auch eine Abdichtung erfolgen.

Mit der Vorrichtung können medizinische Instrumente und nicht implantierte Implantate abgespült oder ausgespült werden, insbesondere medizinischen Instrumente und Implantate mit Hohlräumen, in die der Schlauch eingeführt werden kann. Die Vorrichtung kann aber auch zum freien Verteilen des medizinischen Fluids verwendet werden. Besonders geeignet ist jedoch eine medizinische Anwendung der erfindungsgemäßen Vorrichtung, bei der der Schlauch in eine Kavität eines menschlichen Körpers eingeführt wird und das Fluid zur Behandlung des angrenzenden Gewebes eingesetzt wird.

Ein weiterer überraschender Effekt der vorliegenden Erfindung ist darin zu sehen, dass es mit Hilfe eines Schlauchs mit einer inneren Wandung und einer äußeren Wandung aus unterschiedlichen Materialien gelingt, die in der Schlauchwandung vorhandenen mehreren Öffnungen abhängig vom Druck eines medizinischen Fluids oder abhängig von anderen physikalischen Zustandsgrößen, Wirkungen oder Feldern reversibel zu öffnen und zu schließen, um so temporär ein medizinisches Fluid abzugeben. Durch die Wirkung nur auf die innere Wandung oder nur auf die äußere Wandung des Schlauchs können die mehreren Öffnungen verschlossen oder geöffnet werden. Gleichzeitig werden dabei auf die äußere Wandung keine Kräfte ausgeübt oder eine Verformung der äußeren Form des Schlauchs vermieden. Dadurch bleibt der Schlauch außen formstabil. Eine mechanische Beanspruchung der angrenzenden mit dem Fluid zu behandelnden Oberflächen wird so vermieden. Das hierdurch bereitgestellte Ventil kann so ohne eine Veränderung der äußeren Form des Schlauchs geöffnet und wieder verschlossen werden. Hierdurch kann beispielsweise eine mechanische Reizung eines angrenzenden entzündeten Gewebes verhindert oder zumindest reduziert werden. Insbesondere dann, wenn die mehreren Öffnungen nur dann fluidleitend geöffnet sind, wenn diese durch einen Druck des medizinischen Fluids geöffnet werden, sind die mehreren Öffnungen und damit die Vorrichtung ohne weiteres Einspeisen des medizinischen Fluids geschlossen.

Die Vorrichtung ist kostengünstig vollständig oder weitgehend aus Kunststoff herzustellen und kann so als hygienisches Einmalprodukt zur Verfügung gestellt werden. Die mehreren Öffnungen im Schlauch werden dabei so verschlossen, dass im geschlossenen Zustand keine Hinterschneidungen im Zwischenraum zwischen der äußeren Wandung und der inneren Wandung entstehen, in die Gewebe einwachsen könnte und so ein Entfernen der Vorrichtung beziehungsweise des Schlauchs erschweren würde.

Die Vorrichtung besitzt vorzugsweise eine außerhalb des Patienten zu betätigende Ventilfunktion. Die erfindungsgemäße Vorrichtung kann je nach anatomischer Situation des Implantationsortes oder je nach Tiefe des Hohlraums hinsichtlich ihrer Länge durch einfaches mechanisches Kürzen angepasst werden, ohne dass ein Funktionsverlust eintritt. Dies wird dadurch vereinfacht, dass die Kürzung des Schlauchs auf der proximalen Seite erfolgen kann, wenn die distale Seite bereits innerhalb des zu spülenden Hohlraums angeordnet ist.

Der besondere Vorteil der erfindungsgemäßen Vorrichtung besteht darin, dass der medizinische Anwender jedes beliebige medizinische Fluid mit exakt definiertem Volumen applizieren kann. Bei wirkstoffhaltigen Fluiden können ein oder mehrere pharmazeutische Wirkstoffstoffe in dem Fluid in genau vorbestimmten Konzentrationen eingestellt werden. Dadurch ist es möglich, genau definierte Wirkstoffkonzentrationen in unmittelbarer Nähe der Öffnungen der Vorrichtung zu erreichen und damit zu behandeln. Ein weiterer Vorteil der Vorrichtung ist, dass die mehreren Öffnungen im Schlauch nur während der Applikation geöffnet sind und danach verschlossen werden, so dass kein Blut oder Gewebeflüssigkeit und auch kein sich bildendes Bindegewebe in den Zwischenraum zwischen der inneren Wandung und der äußeren Wandung eindringen kann und dort Hinterschneidungen bilden kann, die beim Entfernen der Vorrichtung reißen und dadurch neue Irritationen des gerade behandelten Gewebes verursachen. Zudem werden Verstopfungen der Vorrichtung, insbesondere der Öffnungen im Schlauch, vermieden.

Ein weiterer Vorteil der erfindungsgemäßen Vorrichtung besteht darin, dass beliebige Kavitäten im humanen Organismus mit pharmazeutischen Fluiden beliebiger Zusammensetzung behandelt werden können. Die Länge und die Gestalt der Vorrichtung sind individuell anpassbar. Es könne pharmazeutische Fluide mit exakt eingestellter Wirkstoffkonzentration appliziert werden. Dadurch ist zum Beispiel auch eine Behandlung von mit multiresistenten Mikroorganismen infizierten Knochenkavitäten durch lokale Applikation von Antibiotika-Gemischen möglich.

Eine beispielhafte erfindungsgemäße Vorrichtung zur lokalen Applikation von Fluiden mit Ventilfunktion ist zusammengesetzt aus
a) einem flexibel verformbaren ersten Schlauch, wobei mindestens zwei Öffnungen in der Mantelfläche des ersten Schlauchs angeordnet sind, welche den Innenraum des ersten Schlauchs mit der Umgebung verbinden, wobei die Summe der Querschnittsflächen der Öffnungen gleich oder kleiner dem Innenquerschnitt des ersten Schlauchs sind,
b) einem Verschlusselement, welches das distale Schlauchendstück des ersten Schlauchs flüssigkeits- und gasdicht verschließt,
c) einem zweiten Schlauch (als die äußere Hülse), der den ersten Schlauch umschließt und der axial auf dem ersten Schlauch verschiebbar ist, wobei der zweite Schlauch am proximalen Schlauchende des ersten Schlauchs angeordnet ist, und
d) einem Konnektor der im proximalen Schlauchende des ersten Schlauchs eingeschraubt oder eingepresst ist, der den ersten Schlauch derart gegen die Innenwand des zweiten Schlauchs presst, dass zwischen dem proximalen Schlauchende des ersten Schlauchs und dem proximalen Schlauchende des zweiten Schlauchs kein Flüssigkeitsaustritt erfolgen kann, wobei der Konnektor flüssigkeitsdurchlässig ist und mit einem diskontinuierlich oder kontinuierlich Wirkstofflösung fördernden Wirkstoffreservoir flüssigkeitsdicht verbindbar oder verbunden ist.

Das Verschlusselement ist beispielsweise zusammengesetzt aus einem ersten rotationssymmetrischer Körper mit Außengewinde, wobei das Außengewinde einen größeren Außendurchmesser besitzt als der Innendurchmesser des Schlauchs, und aus einem zweiten rotationssymmetrischen Körper mit einem Außendurchmesser kleiner oder gleich dem Außendurchmesser des äußeren Schlauchs, wobei die axiale Erstreckung des zweiten rotationssymmetrischen Körpers mindestens 5 mm ist, und wobei der erste rotationssymmetrische Körper axial mit dem zweiten rotationssymmetrischen Körper verbunden ist.

In einer weiteren alternativen Ausgestaltungsform ist ein beispielhaftes Verschlusselement zusammengesetzt aus einem ersten rotationssymmetrischer Körper mit am Umfang umlaufenden Stegen, wobei die Stege einen größeren Außendurchmesser besitzen als der Innendurchmesser des inneren Schlauchs, und aus einem zweiten rotationssymmetrischen Körper mit einem Außendurchmesser kleiner oder gleich dem Außendurchmesser des äußeren Schlauchs, wobei die axiale Erstreckung des zweiten rotationssymmetrischen Körpers mindestens 5 mm ist, und wobei der erste rotationssymmetrische Körper axial mit dem zweiten rotationssymmetrischen Körper verbunden ist.

Vorteilhaft ist eine Kombination der Vorrichtung zum lokalen Applizieren von pharmazeutischen fluiden mit einer Vorrichtung zum kontinuierlichen Auspressen von Fluiden. Diese kombinierte Vorrichtung ist zusammengesetzt aus
a) einem verformbaren Schlauch, wobei mindestens zwei Öffnungen in der Mantelfläche angeordnet sind, welche den Innenraum des Schlauchs mit der Umgebung verbinden, wobei die Summe der Querschnittsflächen der Öffnungen gleich oder kleiner dem Innenquerschnitt des Schlauchs sind,
b) einem Verschlusselement, welches das distale Schlauchendstück des Schlauchs flüssigkeits- und gasdicht verschließt,
c) einer äußeren Hülse, die den Schlauch bereichsweise umschließt und die axial auf dem Schlauch verschiebbar ist, wobei die äußere Hülse am proximalen Schlauchende des Schlauchs angeordnet ist, und
d) einem Konnektor der im proximalen Schlauchende des Schlauchs eingeschraubt oder eingepresst ist, der den Schlauch derart gegen die Innenwand der äußeren Hülse presst, dass zwischen dem proximalen Schlauchende des Schlauchs und dem proximalen Ende der äußeren Hülse kein Flüssigkeitsaustritt erfolgen kann, wobei der Konnektor flüssigkeitsdurchlässig mit einer Vorrichtung zum kontinuierlichen Austragen von pharmazeutischen Fluiden verbunden ist, wobei die Vorrichtung zum kontinuierlichen Austragen von pharmazeutischen Fluiden zusammengesetzt ist aus
e) einem Hohlzylinder in dem sich ein pharmazeutisches Fluid befindet,
f) einem im Hohlzylinder axial verschiebbaren Kolben, der ein Ende des Hohlzylinders verschließt,
g) mindestens einer flüssigkeitsdurchlässigen Austragsöffnung im geschlossenen Kopf des Hohlzylinders,
h) einem Federelement, das mit dem axial verschiebbaren Kolben verbunden ist,
i) wobei das gespannte Federelement den Kolben in Richtung der Austragsöffnung bewegt, und
j) das pharmazeutische Fluid im Hohlzylinder durch die Austragsöffnung und durch den Konnektor in das proximale Schlauchende des Schlauchs einpresst.

Vorteilhaft ist es, wen ein Ventilelement zwischen der Austragsöffnung und dem Konnektor angeordnet ist. Damit kann der Volumenstrom des pharmazeutischen Fluids reguliert werden. Mit dieser Vorrichtung ist es möglich, über einen Zeitraum von Stunden bis mehreren Tagen das pharmazeutische Fluide lokal zu applizieren, ohne das aufwendige elektrisch betriebene Pumpsysteme notwendig sind.

Im Folgenden werden weitere Ausführungsbeispiele der Erfindung anhand von achtzehn schematisch dargestellten Figuren erläutert, ohne jedoch dabei die Erfindung zu beschränken. Dabei zeigt:
Figur 1: eine schematische perspektivische Ansicht einer beispielhaften ersten erfindungsgemäßen Vorrichtung zur lokalen Applikation eines Fluids;
Figur 2: eine schematische perspektivische Ansicht auf die Vorrichtung nach Figur 1, bei der ein Schlauch der Vorrichtung nicht mit einem Konnektor verbunden ist;
Figur 3: eine schematische perspektivische Ansicht auf die Vorrichtung nach Figur 2, bei der der Schlauch gekürzt wurde;
Figur 4: eine schematische perspektivische Ansicht auf die Vorrichtung nach Figur 3, bei der der gekürzte Schlauch noch nicht mit dem Konnektor verbunden wurde;
Figur 5: eine schematische perspektivische Ansicht auf die Vorrichtung nach Figur 4, bei der der gekürzte Schlauch mit dem Konnektor verbunden wurde;
Figur 6: eine schematische perspektivische Teilschnittansicht einer Fördereinrichtung für den Behälter einer Vorrichtung nach den Figuren 1 bis 5;
Figur 7: eine schematische Teilschnittaufsicht der Fördereinrichtung nach Figur 6 im gespannten Zustand;
Figur 8: eine schematische Teilschnittaufsicht der Fördereinrichtung nach Figur 6 im entspannten Zustand;
Figur 9: vier schematische Querschnittansichten von distalen Schlauchteilstücken zweier erfindungsgemäßer Vorrichtungen mit geöffneten Öffnungen;
Figur 10: vier schematische Querschnittansichten der distalen Schlauchteilstücken der beiden Vorrichtungen nach Figur 9 mit geschlossenen Öffnungen;
Figur 11: eine Ausschnittvergrößerung der Figur 2;
Figur 12: eine schematische perspektivische Querschnittansicht der Vorrichtung nach Figur 5, bei der der gekürzte Schlauch mit dem Konnektor verbunden wurde;
Figur 13: eine schematische perspektivische Teilschnittansicht der Vorrichtung nach den Figuren 1 bis 5 und 11 und 12, während des Auspressens eines medizinischen Fluids;
Figur 14: eine schematische perspektivische Teilschnittansicht der Vorrichtung nach Figur 5 als Ausschnittvergrößerung im Bereich des Konnektors;
Figur 15: eine schematische perspektivische Teilschnittansicht der Vorrichtung nach den Figuren 1 bis 5 und 11 bis 14 als Ausschnittvergrößerung im Bereich des distalen Schlauchendes des Schlauchs;
Figur 16: eine schematische Seitenansicht als Ausschnittvergrößerung einer erfindungsgemäßen Vorrichtung, bei der der Konnektor von dem Behälter und dem Schlauch getrennt ist;
Figur 17: eine schematische Teil-Querschnittansicht im Bereich eines Versiegelungselements einer erfindungsgemäßen Vorrichtung als Ausschnittvergrößerung; und
Figur 18: eine schematische Teil-Querschnittansicht im Bereich eines Versiegelungselements einer weiteren erfindungsgemäßen Vorrichtung als Ausschnittvergrößerung.

In den Figuren und in der folgenden Beschreibung zu den anhand der Figuren erläuterten Ausführungsbeispiele der vorliegenden Erfindung werden teilweise für unterschiedliche Ausführungsbeispiele die gleichen Bezugszeichen bei gleichen oder gleichartigen Teilen verwendet, um die Vergleichbarkeit der Ausführungsbeispiele und die Lesbarkeit zu vereinfachen.

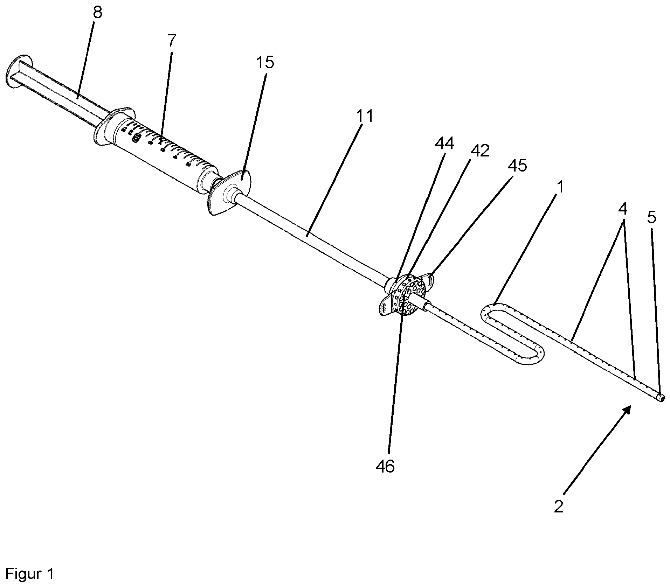

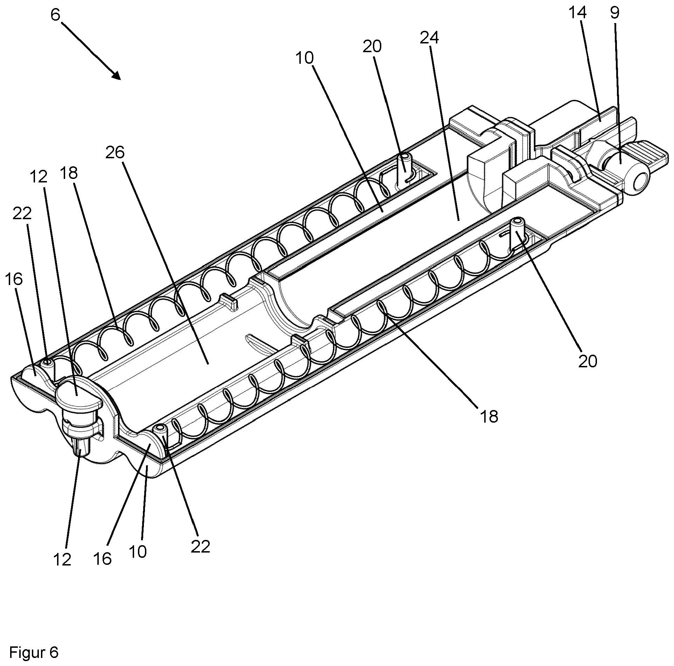

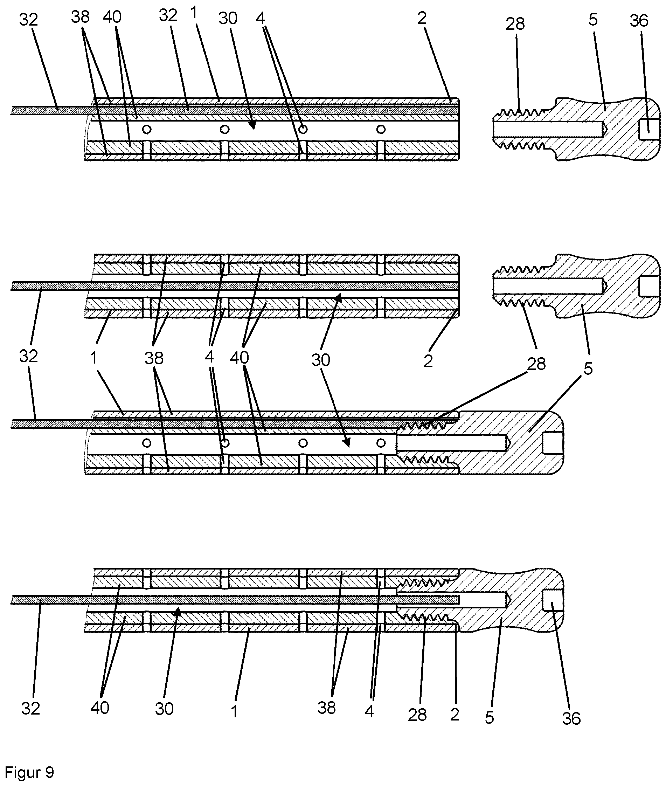

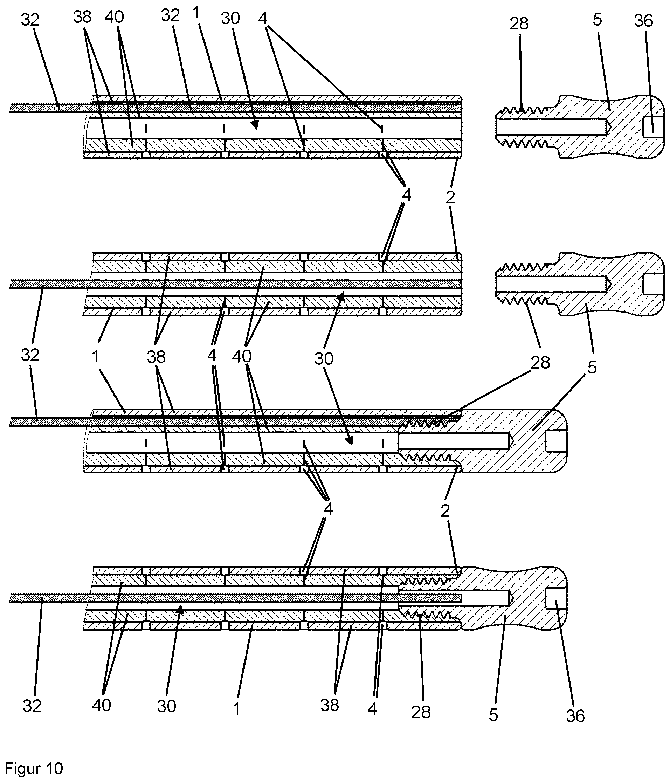

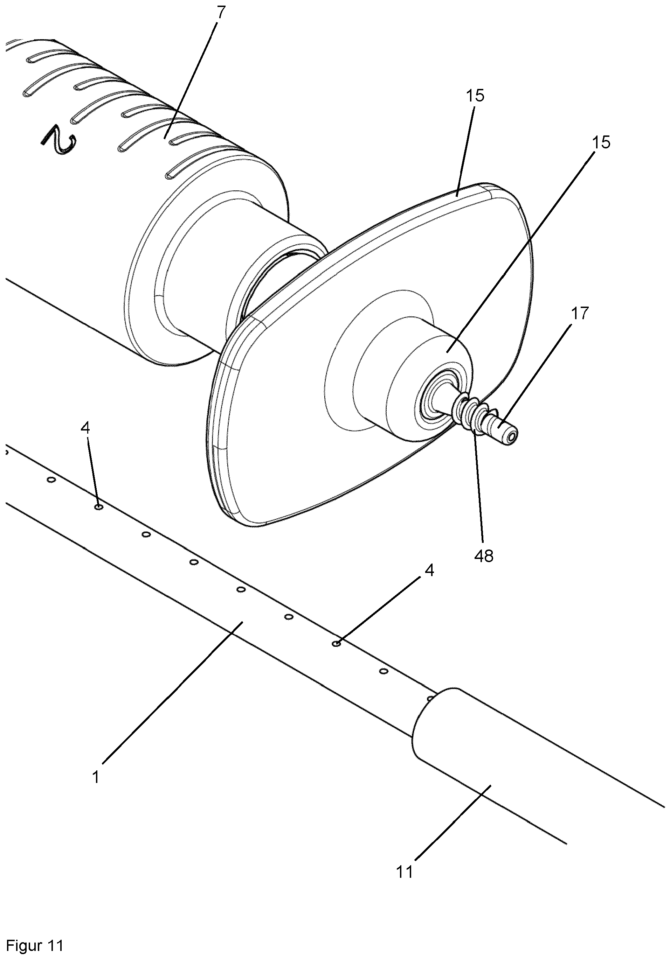

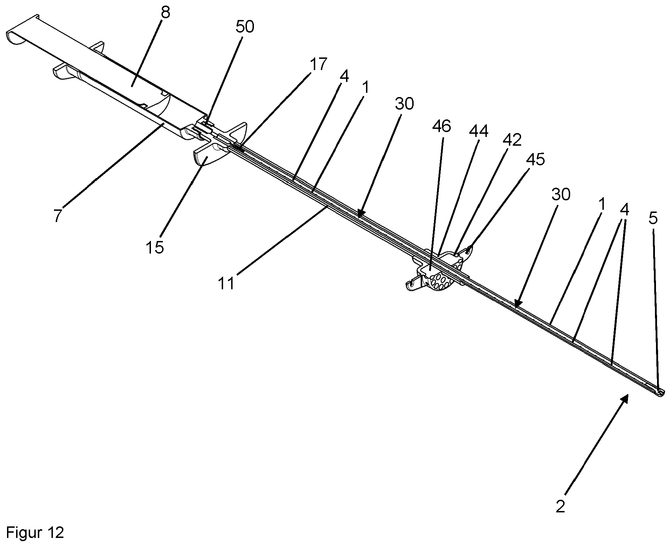

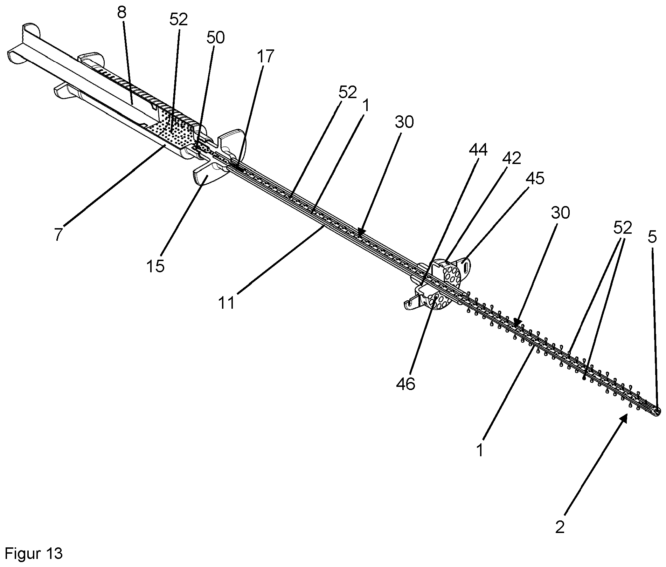

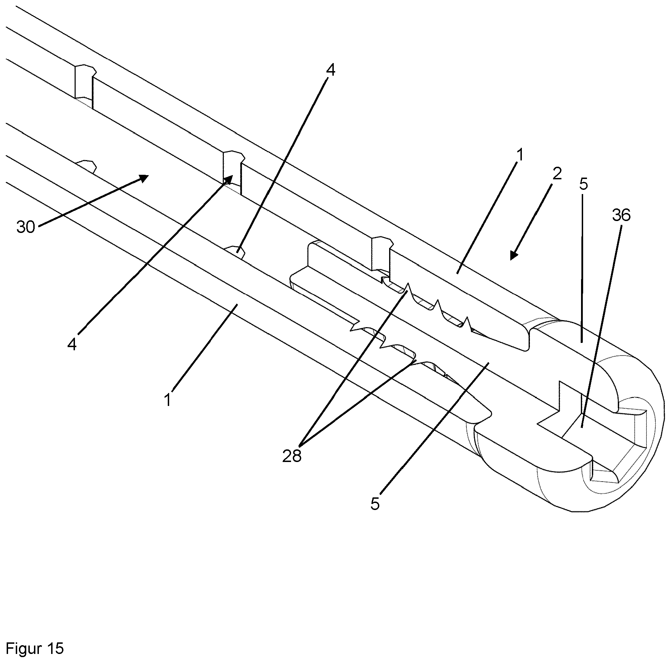

Die Figuren 1 bis 5 und 11 bis 17 zeigen eine erste beispielhafte erfindungsgemäße Vorrichtung und Teile davon in unterschiedlichen Darstellungen. Die Figuren 6 bis 8 zeigen eine Fördereinrichtung zum Auspressen eines medizinischen Fluids einer erfindungsgemäßen Vorrichtung. Die Figuren 9 und 10 zeigen offene und geschlossene distale Schlauchendstücke als Teile von zwei unterschiedlichen erfindungsgemäßen Ausführungsbeispielen. Die Figuren 11 und 14 bis 19 zeigen Ausschnittvergrößerungen von erfindungsgemäßen Vorrichtungen. In den Figuren 12 und 13 sind eine Querschnittansicht und eine Teilschnittansicht der ersten erfindungsgemäßen Vorrichtung gezeigt.

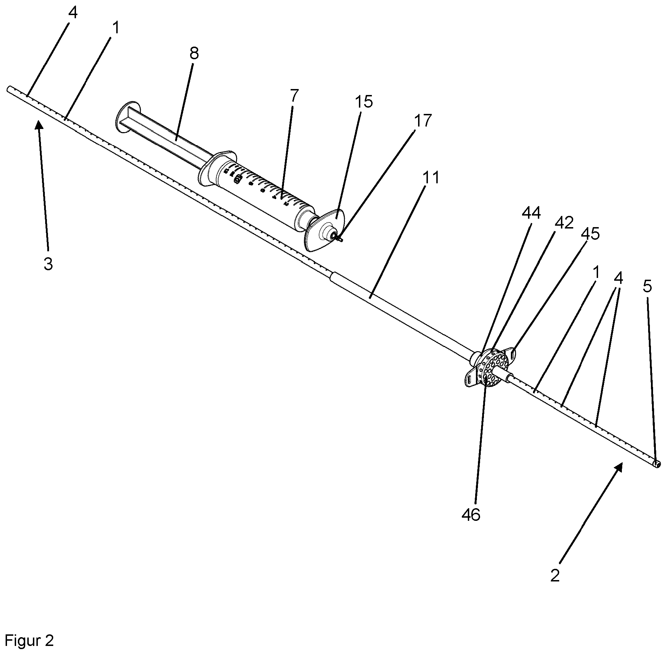

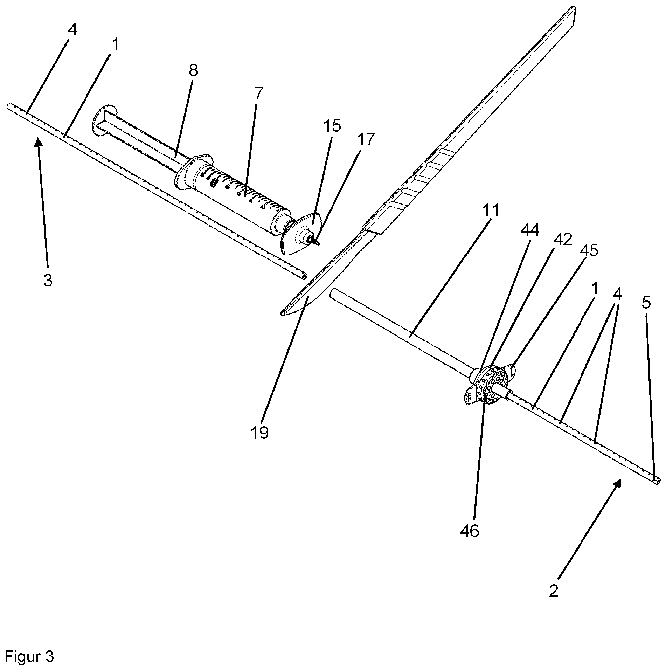

Die erste beispielhafte erfindungsgemäße Vorrichtung, die in den Figuren 1 bis 5 und 11 bis 17 gezeigt ist, hat an der vorderen distalen Seite (in den Figuren 1, 4, 5, 12 und 13 rechts unten, in den Figuren 2 und 3 unten) einen Schlauch 1. Der Schlauch 1 kann ein distales Schlauchende 2 und ein dem distalen Schlauchende 2 gegenüberliegendes proximales Schlauchende 3 (siehe Figuren 2 und 3) aufweisen. In dem Schlauch 1 können mehrere durch die Schlauchwandung gehende Öffnungen 4 angeordnet sein, die bis in eine innere Leitung 30 (in den Figuren 1 bis 5 nicht zu sehen, aber in den Figuren 12 bis 15) des Schlauchs 1 reichen. Der Schlauch 1 kann plastisch verformbar sein, so dass die Form des Schlauchs 1 an die Form eines zu spülenden Hohlraums angepasst werden kann.

Auf dem Schlauch 1 kann eine axial auf dem Schlauch 1 verschiebbare äußere Hülse 11 in Form eines im Vergleich zum Schlauch 1 kürzeren Schlauchstücks oder eines im Vergleich zum Schlauch 1 kürzeren Rohrs angeordnet sein. Die äußere Hülse 11 kann einen zum Außendurchmesser des Schlauchs 1 passenden Innendurchmesser aufweisen, so dass die äußere Hülse 11 diejenigen Öffnungen 4 in der Schlauchwand abdeckt und fluiddicht verschließt, über denen sie angeordnet ist. Damit wird sichergestellt, dass ein medizinisches Fluid 52 (siehe Figur 13), das mit einer solchen Vorrichtung appliziert wird, nur innerhalb des zu spülenden Hohlraums aus der Vorrichtung austritt.

Der Schlauch 1 kann an seinem distalen Schlauchende 2 mit einem Verschlusselement 5 fluiddicht und druckdicht verschlossen sein. Der Schlauch 1 weist eine innere Wandung 40 und eine äußere Wandung 38 auf (in den Figuren 1 bis 5 und 11 bis 17 nicht zu sehen, aber analog den Figuren 9 und 10 aufgebaut), wobei die äußere Wandung 38 die innere Wandung 40 umschließt, bevorzugt koaxial umschließt. Die Öffnungen 4 reichen durch die äußere Wandung 38 und die innere Wandung 40. Im Inneren des Schlauchs 1 ist die innere Leitung 30 vorzugsweise durch die innere Wandung 40 begrenzt. Die Öffnungen 4 können die innere Leitung 30 mit der Umgebung des Schlauchs 1 flüssigkeitsdurchlässig verbinden. Die innere Wandung 40 kann aus einem elastisch verformbaren Material wie einem gummielastischen Polymer, insbesondere Polyurethan, bestehen. Die äußere Wandung 38 kann aus einem nicht gummielastischen thermoplastischen Polymer, insbesondere aus Polyamid, bestehen. Dadurch ist die innere Wandung 40 elastisch verformbar, während die äußere Wandung 38 gegenüber einer radialen Ausdehnung des Schlauchs 1 weitgehend formstabil ist. Das Material für die äußere Wandung 38 kann so gewählt werden, dass eine Verformung der Längsachse des Schlauchs 1 möglich ist, während eine radiale Ausdehnung des Schlauchs 1 aufgrund eines inneren Drucks in der inneren Leitung 30 nicht möglich ist oder nur maximal eine fünfprozentige relative axiale Dehnung möglich ist.

Die Öffnungen 4 können durch die innere Wandung 40 des Schlauchs 1 gestochen sein, so dass das Material der inneren Wandung 40 nicht ausgestanzt wird. Ähnlich wie bei gummielastischen Membranen für Behälter zum Aufziehen von Spritzen können sich dadurch die Öffnungen 4 in der inneren Wandung 40 verschließen (siehe Figur 10). Wenn in der inneren Leitung 30 ein Druck auf ein Fluid darin ausgeübt wird, öffnet dieser Druck die Öffnungen 4 in den inneren Wandungen 40 des Schlauchs 1 (siehe Figur 9) und das Fluid kann aus den Öffnungen 4 austreten, so wie das in Figur 13 durch die austretenden Tropfen des medizinischen Fluids 52 angedeutet ist.

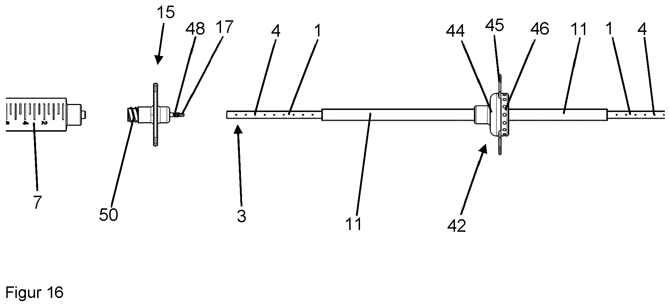

Die Öffnungen 4 können radial verteilt und entlang der gesamten Länge des Schlauchs 1 verteilt sein. Die in dem Ausführungsbeispiel angedeuteten Öffnungen 4 in vier axialen Richtungen sind dabei nur beispielhaft zu verstehen. Der Schlauch 1 kann auch an die Größe des zu spülenden Hohlraums angepasst werden, indem er an einer proximalen Seite durch abschneiden gekürzt wird (siehe Figur 3). Mit einem Konnektor 15 kann das neue geschnittene proximale Schlauchende fluiddicht mit einem Behälter 7 zur Aufnahme des medizinischen Fluids 52 verbunden werden. Hierzu kann ein Anschluss 17 des Konnektors 15 in die innere Leitung 30 eingesteckt oder eingeschraubt werden. An dem Anschluss 17 des Konnektors 15 kann hierzu Außengewinde 48 angeordnet sein (siehe Figur 11 und 16), das in das proximale Schlauchende des Schlauchs 1 geschraubt werden kann. Auf der dem Anschluss 17 gegenüberliegenden proximalen Seite des Konnektors 15 kann ein Stutzen 50 mit einem Außengewinde (siehe Figuren 12 und 13 und 16) oder mit einem Luer-Lock-Anschluss angeordnet sein, mit dem der Konnektor 15 fluiddicht mit dem Behälter 7 verbindbar ist.

An der proximalen Seite der Vorrichtung kann eine Fördereinrichtung 6 (siehe die Figuren 6 bis 8) angeordnet sein oder werden. In die Fördereinrichtung 6 kann der Behälter 7 in Form einer Spritze mit einem Kolben 8 zum Auspressen des Inhalts der Spritze eingesetzt werden beziehungsweise eingesetzt sein. Der Kolben 8 kann in axialer Richtung in der Spritze verschiebbar angeordnet sein und fluiddicht gegen die Innenwand des Behälters 7 abgedichtet sein. Die Fördereinrichtung 6 kann ein Gehäuse 10 aus einem Plastik aufweisen, das das Innere der Fördereinrichtung 6 vollständig oder teilweise nach außen verschließen kann. Ein Sicherungsbolzen 12 kann in eine Öffnung am proximalen Ende des Gehäuses 10 eingesteckt sein.

Auf der distalen Seite der Fördereinrichtung 6 kann eine Halterung 14 zur Befestigung des Konnektors 15 mit dem Schlauch 1 angeordnet sein. Hierzu kann der Konnektor 15 eine Halterungsscheibe aufweisen, die in die Halterung 14 eingreifen kann.

In der Fördereinrichtung 6 kann eine Förderplatte 16 zum Einpressen des Kolbens 8 in den Behälter 7 angeordnet sein. Mit dem Sicherungsbolzen 12 kann die Förderplatte 16 gegen das Gehäuse 10 arretiert werden. Hierzu kann eine Öse an der proximalen Seite der Förderplatte 16 aus dem Gehäuse 10 der Fördereinrichtung 6 ragen und durch Einstecken des Sicherungsbolzens 12 die Förderplatte 16 gegen das Gehäuse 10 arretiert werden. Die Förderplatte 16 kann von zwei gespannten Federn 18 angetrieben werden. Die beiden Federn 18 stellen ein Energiespeicherelement dar, in denen zumindest die Energie gespeichert ist, die zum Auspressen eines medizinischen Fluids 52 aus dem Behälter 7 und durch den Schlauch 1 und durch die Öffnungen 4 des Schlauchs 1 notwendig ist.

Die Federn 18 können an ihren distalen Enden mit Stiften 20 am Gehäuse 10 befestigt sein. An ihren proximalen Enden können die Federn 18 mit Stiften 22 an der Förderplatte 16 befestigt sein. Die Federn 18 können so zwischen den Stiften 20 und den Stiften 22 gespannt sein.

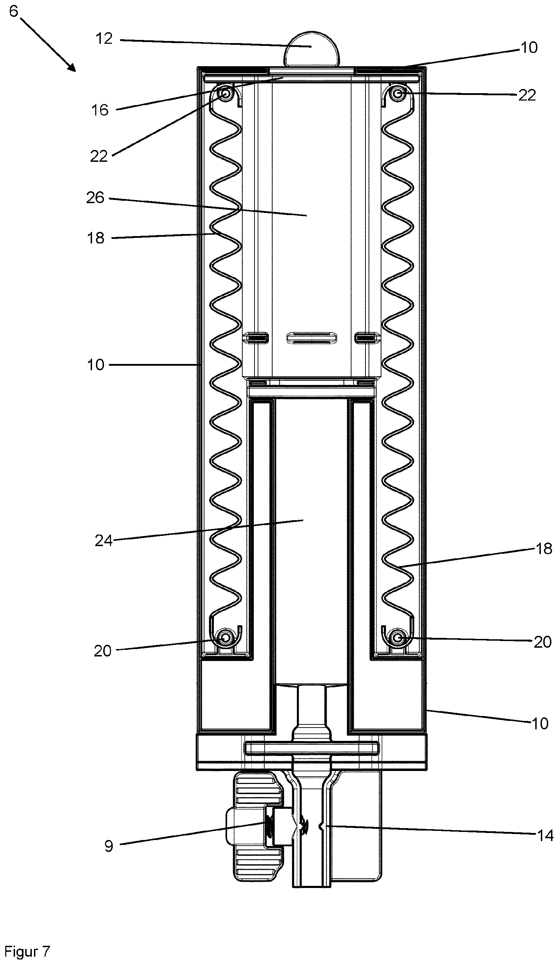

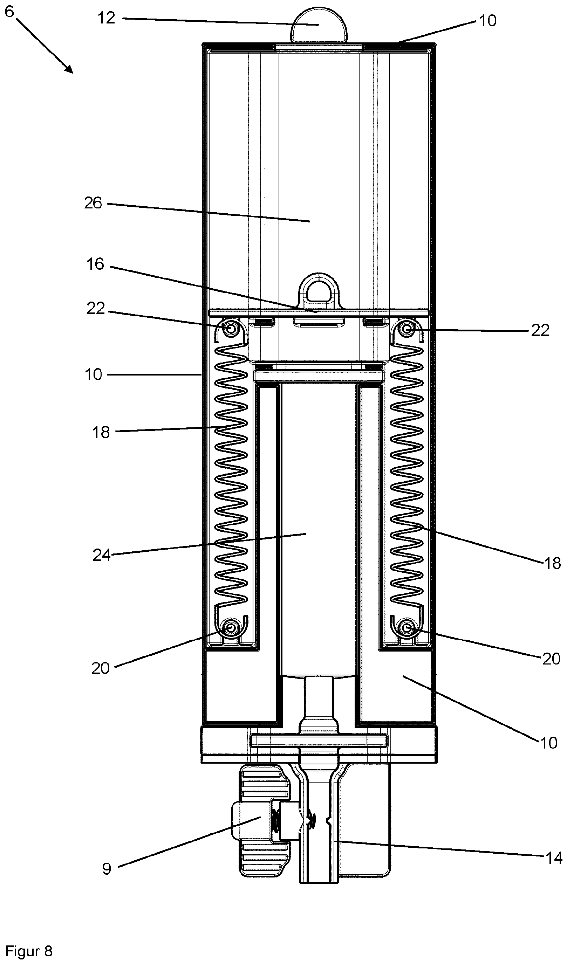

Im Inneren des Gehäuses 10 kann eine Aufnahme 24 für den Behälter 7 und ein Hubraum 26 für den Kolben 8 ausgeformt sein. Der Behälter 7 kann durch die Form der Aufnahme 24 in der Fördereinrichtung 6 fixiert werden. Die Förderplatte 16 kann auf diese Weise von den Federn 18 vom proximalen Ende bis zum distalen Ende des Hubraums 26 gezogen werden (siehe Figuren 7 und 8). Der Kolben 8 kann in der Fördereinrichtung 6 mit der Förderplatte 16 angetrieben durch die Federn 18 in den Behälter 7 gepresst werden, wenn der Sicherungsbolzen 12 entfernt wurde und das Ventilelement 9 geöffnet ist. Dadurch kann ein in dem Behälter 7 enthaltenes medizinisches Fluid 52 aus dem Behälter 7 und durch den Schlauch 1 und die Öffnungen 4 des Schlauchs 1 ausgepresst werden. Mit dem auf das medizinische Fluid 52 wirkenden Druck können dabei die Öffnungen 4 in der inneren Wandung des Schlauchs 1 geöffnet werden.

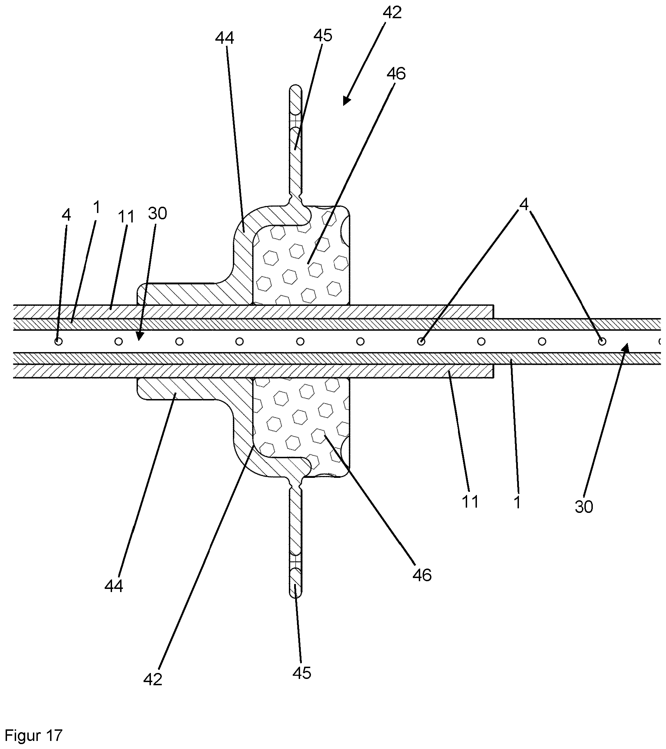

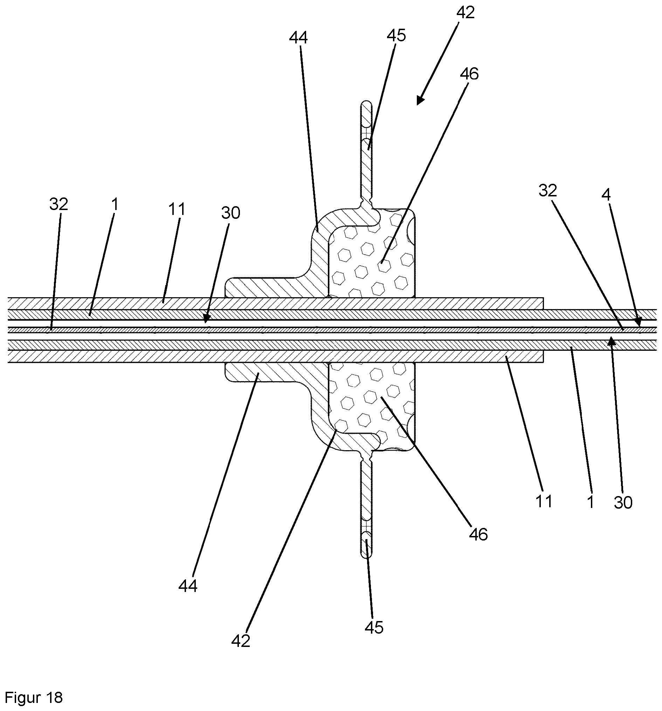

Die in den Figuren 9 und 10 gezeigten Schläuche 1 können ohne weiteres in der nach den Figuren 1 bis 5 und 11 bis 17 gezeigten Vorrichtung angewendet werden und so die Figuren 9 und 10 als Detailzeichnungen des ersten Ausführungsbeispiels verstanden werden. Die Varianten der Schläuche 1 nach den Figuren 9 und 10 und 18 unterscheiden sich durch die Anordnung eines Metalldrahts 32, der entweder in einer inneren Leitung 30 des Schlauchs 1 angeordnet sein kann oder in der Schlauchwand. Der Aufbau nach Figur 18 entspricht dem nach Figur 17, bis auf den Metalldraht 32, der in der inneren Leitung 30 des Schlauchs 1 angeordnet ist, um eine plastische Verformung des Schlauchs 1 zu ermöglichen. Wenn der Metalldraht 32 in der inneren Leitung 30 angeordnet ist, kann der Metalldraht 32 einen geringeren Durchmesser aufweisen, als der Innendurchmesser der inneren Leitung 30, damit die innere Leitung 30 einen ausreichenden freien Querschnitt zur Leitung des medizinischen Fluids 52 behält.

Der Schlauch 1 kann in allen Fällen an seinem distalen Schlauchende 2 mit einem Verschlusselement 5 verschließbar sein. Das Verschlusselement 5 kann einen vorstehenden zylindrischen Fortsatz mit einem Außengewinde 28 aufweisen. Mit dem Außengewinde 28 kann das Verschlusselement 5 in die offene innere Leitung 30 des Schlauchs 1 eingeschraubt werden. Das Verschlusselement 5 besteht vorzugsweise aus Metall. Das Außengewinde 28 kann sich ein passendes Innengewinde in die Innenwand des Schlauchs 1 schneiden. Dadurch kann der Schlauch 1 an seinem distalen Schlauchende 2 flüssigkeitsdicht und druckdicht verschlossen werden. In dem distalen Kopf des Verschlusselements 5 kann ein Innensechskant 36 angeordnet sein oder es kann ein anderer Schraubkopf vorgesehen sein, um das Verschlusselement 5 bequemer in den Schlauch 1 einschrauben zu können.

Analog dem ersten Ausführungsbeispiel nach den Figuren 1 bis 5 und 11 bis 17 weist der Schlauch 1 eine äußere Wandung 38 und eine innere Wandung 40 auf (siehe Figuren 9 und 10). Die äußere Wandung 38 umschließt die innere Wandung 40 vorzugsweise vollständig. Die Materialien zur Ausbildung der äußeren Wandung 38 und der inneren Wandung 40 können dabei analog dem ersten Ausführungsbeispiel so gewählt werden, dass die äußere Wandung 38 formstabil ist und die innere Wandung 40 gummielastisch ist. Mehrere Öffnungen 4 reichen durch die äußere Wandung 38 und die innere Wandung 40 bis zur inneren Leitung 30 des Schlauchs 1.

Wenn kein Druck eines medizinischen Fluids in der inneren Leitung 30 des Schlauchs 1 wirkt, kann das Material der inneren Wandung 40 entspannen. Die Öffnungen 4 werden dadurch in der inneren Wandung 40 fluiddicht verschlossen (siehe Figur 10). Die Öffnungen 4 sind in Figur 10 in der inneren Wandung 40 als Schlitze zu erkennen. Durch einen Druck auf das medizinische Fluid können die Öffnungen 4 in der inneren Wandung 40 geöffnet werden. Dabei wird der Schlauch 1 nicht radial ausgedehnt, da die äußere Wandung 38 die Kräfte aufnehmen kann. Das medizinische Fluid kann durch die geöffneten Öffnungen 4 austreten.

Mit Hilfe des Metalldrahts 32 kann der Schlauch 1 bei beiden Ausführungen plastisch verformt werden und hält seine Form.

Bei dem ersten Ausführungsbeispiel kann ein Versiegelungselement 42 auf den Schlauch 1 gesteckt sein. Bevorzugt kann das Versiegelungselement 42 auf dem Schlauch 1 axial (bezogen auf die zylindrische Schlauchachse) verschiebbar sein, insbesondere zusammen mit der äußeren Hülse 11 (siehe auch Figur 17). Das Versiegelungselement 42 kann fest mit der äußeren Hülse 11 verbunden sein. Gemäß einer bevorzugten Ausführung ist das Versiegelungselement 42 im Bereich des distalen Endes der äußeren Hülse 11 angeordnet. Dann ist für den Anwender erkennbar, wo das zu implantierende distale Teilstück des Schlauchs 1 endet beziehungsweise wo das angrenzende nicht zu implantierende proximale Teilstück des Schlauchs 1 beginnt. Zudem kann die äußere Hülse 11 so bequem mit dem Versiegelungselement 42 auf dem Schlauch 1 verschoben werden.

Das Versiegelungselement 42 kann hülsenförmig geformt sein und eine äußere Hülsenform 44 mit zwei Halterungsflügeln 45 und eine distale Schwammhülse 46 aufweisen. Die Schwammhülse 46 kann mit einer antibiotischen und/oder desinfizierenden Lösung getränkt sein. Das Versiegelungselement 42 kann auf eine Eintrittsöffnung eines Körpers geschoben werden und dadurch einen Keimeintritt in die Eintrittsöffnung verhindern. Mit Hilfe der Halterungsflügel 45 kann das Versiegelungselement 42 auf der Haut eines Patienten oder an einem Implantat befestigt werden.

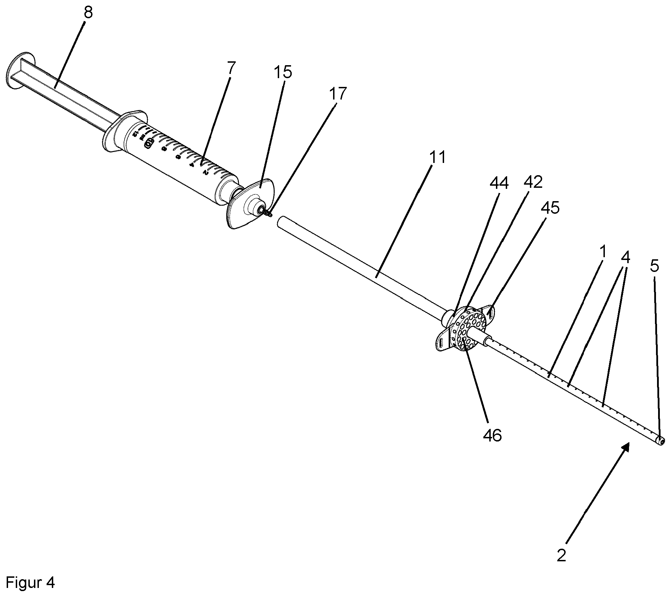

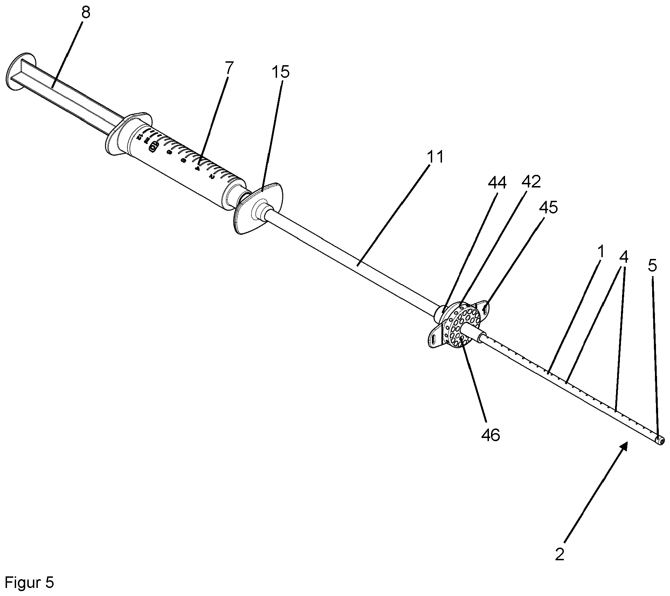

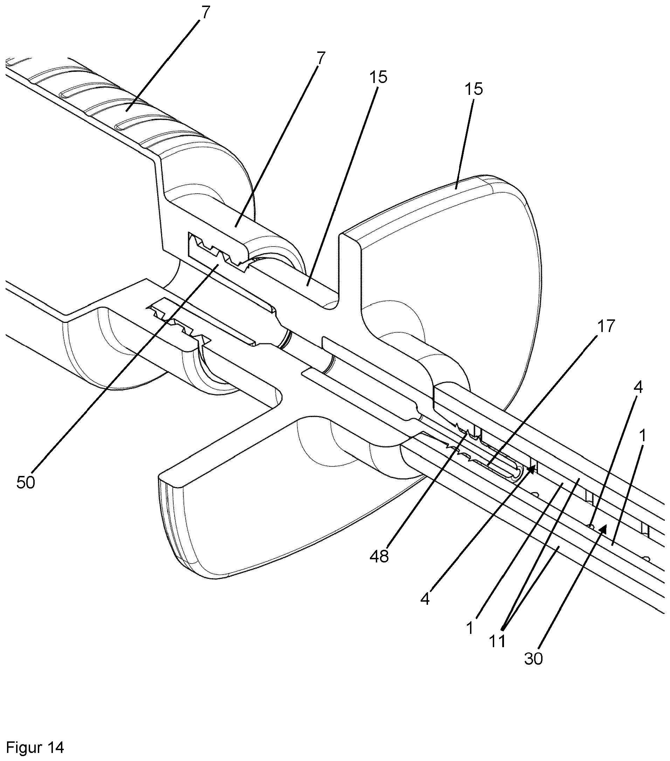

Die Verwendung der erfindungsgemäßen Vorrichtung erfolgt in der Weise, dass vom medizinischen Personal die Länge der zu behandelnden Kavität oder des medizinischen Implantats bestimmt wird. Dann wird die äußere Hülse 11 in Form des äußeren Schlauchs auf dem Schlauch 1 solange in Richtung des distalen Schlauchendes 2 geschoben, bis das distale Ende der äußeren Hülse 11 am Rand der zu behandelnden Kavität oder des medizinischen Implantats angelangt ist (siehe Figur 2). Danach wird der aus dem proximalen Ende der äußeren Hülse 11 herausstehende Schlauch 1 direkt neben dem proximalen Ende der äußeren Hülse 11 abgeschnitten (siehe Figur 3). Das neue proximale Schlauchende des Schlauchs 1 wird durch Einschrauben oder Einpressen des Konnektors 15 verschlossen (siehe den Übergang von Figur 4 auf Figur 5), wobei der Konnektor 15 die Außenseite des Schlauchs 1 gegen die Innenseite der äußeren Hülse 11 presst (siehe Figuren 13 und 14). Der Konnektor 15 ist flüssigkeitsdurchlässig mit dem Behälter 7 enthaltend das Fluidreservoir verbunden (siehe Figur 13) oder ist damit verbindbar. Anschließend kann das medizinische Fluid 52 mit dem Kolben 8 aus dem Behälter 7 durch den Konnektor 15 und durch die innere Leitung 30 des Schlauchs 1 aus den freiliegenden Öffnungen 4 auf der distalen Seite des Schlauchs 1 ausgepresst werden (siehe Figur 13). Die äußere Hülse 11 verhindert dabei, dass das medizinische Fluid 52 aus den Öffnungen 4 an der proximalen Seite des Schlauchs 1 austreten kann, die außerhalb der behandelnden Kavität oder des medizinischen Implantats angeordnet ist.

Als das zu applizierende medizinische Fluid 52 kann je nach Anwendung beispielsweise eine desinfizierende Flüssigkeit oder eine wässrige Lösung umfassend wenigstens ein Antibiotikum und/oder wenigstens ein Antimykotikum verwendet werden. Ferner kann das medizinische Fluid 52 auch wenigstens ein Zytostatikum und/oder wenigstens ein Chemotherapeutikum enthalten.

Für eine medizinische Anwendung der erfindungsgemäßen Vorrichtungen können der Schlauch 1 und vorzugsweise auch die Verschlusselemente 5 aus biokompatiblen Materialien aufgebaut werden, in denen Röntgenopaker enthalten sind, so dass die Lage des Schlauchs 1 und gegebenenfalls des Verschlusselements 5 mit bildgebenden Röntgenverfahren bestimmbar ist.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Schlauch
- 2: Distales Schlauchende
- 3: Proximales Schlauchende
- 4: Öffnung
- 5: Verschlusselement
- 6: Fördereinrichtung
- 7: Behälter
- 8: Kolben
- 9: Ventilelement
- 10: Gehäuse
- 11: Äußere Hülse
- 12: Verschlussbolzen
- 14: Halterung
- 15: Konnektor
- 16: Förderplatte
- 17: Anschluss
- 18: Feder
- 19: Skalpell
- 20: Stift
- 22: Stift
- 24: Aufnahme für Behälter
- 26: Hubraum für Kolben
- 28: Außengewinde
- 30: Innere Leitung des Schlauchs
- 32: Metalldraht
- 36: Innensechskant
- 38: Äußere Wandung
- 40: Innere Wandung
- 42: Versiegelungselement
- 44: Hülsenform
- 45: Halterungsflügel
- 46: Schwammhülse
- 48: Außengewinde
- 50: Stutzen
- 52: Medizinisches Fluid

## Patentansprüche

1. Vorrichtung zur lokalen Applikation eines medizinischen Fluids (52) aufweisend einen Schlauch (1), der flexibel verformbar ist und der eine Schlauchwand aufweist, wobei der Schlauch (1) mehrere Öffnungen (4) in der Schlauchwand aufweist, wobei die mehreren Öffnungen (4) eine innere Leitung (30) des Schlauchs (1) mit der Umgebung des Schlauchs (1) verbinden und wobei der Schlauch (1) an einem distalen Schlauchende (2) des Schlauchs (1) verschlossen ist,
wobei ein proximales Schlauchende (3) des Schlauchs (1) derart mit einem Behälter (7) für das medizinische Fluid (52) flüssigkeitsdurchlässig verbunden ist oder verbindbar ist, dass das medizinische Fluid (52) aus dem Behälter (7) durch das proximale Schlauchende (3) des Schlauchs (1) in die innere Leitung (30) des Schlauchs (1) drückbar ist und durch die mehreren Öffnungen (4) in die Umgebung des Schlauchs (1) herausdrückbar ist,
wobei die Vorrichtung eine äußere Hülse (11) zum fluiddichten Verschließen eines Teils der mehreren Öffnungen (4) aufweist, wobei die äußere Hülse (11) axial verschiebbar um den Schlauch (1) herum angeordnet ist und wobei die äußere Hülse (11) kürzer ist als der Schlauch (1), so dass die nicht zum verschlossenen Teil der mehreren Öffnungen (4) gehörenden distalen Öffnungen frei liegen, wobei die Schlauchwand eine äußere Wandung (38) aus einem ersten Material aufweist, die radial außen liegend angeordnet ist, und die Schlauchwand eine innere Wandung (40) aus einem zweiten Material aufweist, die radial innen liegend angeordnet ist und die die innere Leitung (30) des Schlauchs (1) begrenzt, wobei
die äußere Wandung (38) und die innere Wandung (40) miteinander fest verbunden sind, bevorzugt vollflächig miteinander verbunden sind.

2. Vorrichtung nach Anspruch 1, **dadurch gekennzeichnet, dass**
die mehreren Öffnungen (4) in der äußeren Wandung (38) der Schlauchwand unabhängig vom Druck des medizinischen Fluids (52) offen sind, während die mehreren Öffnungen (4) in der inneren Wandung (40) der Schlauchwand ohne Druckbeaufschlagung durch das medizinische Fluid (52) verschlossen sind und durch einen Druck auf das medizinische Fluid (52) flüssigkeitsdurchlässig zu öffnen sind.

3. Vorrichtung nach Anspruch 1 oder 2, **dadurch gekennzeichnet, dass**
die äußere Wandung (38) der Schlauchwand einen über die innere Wandung (40) der Schlauchwand vermittelten Druck des medizinischen Fluids (52) in der inneren Leitung (30) aufnimmt, ohne sich dabei radial um mehr als 5% auszudehnen, bevorzugt ohne sich dabei radial um mehr als 1% auszudehnen.

4. Vorrichtung nach einem der vorangehenden Ansprüche, **dadurch gekennzeichnet, dass**
das erste Material eine größere Shore A-Härte hat als das zweite Material, wobei bevorzugt das erste Material eine Shore A-Härte von mehr als 60 und das zweite Material eine Shore A-Härte von weniger als 60 hat, und/oder die mehreren Öffnungen (4) in der inneren Wandung (40) bei Druckbeaufschlagung mit einem hydrostatischen Druck von 500 kPa durch das medizinische Fluid (52) einen um den Faktor zwei oder mehr größeren freien Querschnitt aufweisen als ohne Druckbeaufschlagung, wobei vorzugsweise
die mehreren Öffnungen (4) in der inneren Wandung (40) oder der äußeren Wandung (38) schlitzförmig geformt sind.

5. Vorrichtung nach einem der vorangehenden Ansprüche, **dadurch gekennzeichnet, dass**
die mehreren Öffnungen (4) im Schlauch (1) in der äußeren Wandung (38) einen Durchmesser von maximal 500 µm, bevorzugt von maximal 250 µm und besonders bevorzugt von maximal 100 µm haben, und/oder
der Schlauch (1) aus einem koaxialen Koextrudat gebildet ist, wobei die innere Wandung (40) aus einem gummielastischen Polymer, insbesondere Polyurethan oder einem schwach vernetzten Polymer, besteht und die äußere Wandung (38) aus einem nicht gummielastischen thermoplastischen Polymer oder aus einem stark vernetzten Polymer besteht, insbesondere aus Polyamid besteht.

6. Vorrichtung nach einem der vorangehenden Ansprüche, **dadurch gekennzeichnet, dass**
die Vorrichtung ein Verschlusselement (5) aufweist, mit dem der Schlauch (1) an dem distalen Schlauchende (2) des Schlauchs (1) flüssigkeitsdicht verschlossen ist, wobei die innere Leitung (30) des Schlauchs (1) an einer proximalen Öffnung im proximalen Schlauchende (3) des Schlauchs (1) beginnt und an einer distalen Öffnung im distalen Schlauchende (2) des Schlauchs (1) endet, wobei vorzugsweise die distale Öffnung des Schlauchs (1) durch das Verschlusselement (5) verschlossen ist.

7. Vorrichtung nach einem der vorangehenden Ansprüche, **dadurch gekennzeichnet, dass**
die Vorrichtung den Behälter (7) für das medizinische Fluid (52) aufweist, wobei bevorzugt der Behälter (7) einen Hohlzylinder mit einem axial im Hohlzylinder verschiebbaren Kolben (8) aufweist, der ein erstes Ende des Hohlzylinders verschließt, wobei der Hohlzylinder an einem dem ersten Ende gegenüberliegenden Ende eine Austragsöffnung aufweist, die mit dem proximalen Schlauchende (3) des Schlauchs (1) verbunden oder verbindbar ist, bevorzugt über ein manuell bedienbares Ventilelement (9) zur Regulierung der Strömungsgeschwindigkeit des medizinischen Fluids (52) mit dem proximalen Schlauchende (3) des Schlauchs (1) verbunden oder verbindbar ist, wobei vorzugsweise
in dem Behälter (7) ein medizinisches Fluid (52), insbesondere ein pharmazeutisches Fluid, enthalten ist.

8. Vorrichtung nach einem der vorangehenden Ansprüche, **dadurch gekennzeichnet, dass**
die Vorrichtung eine Fördereinrichtung (6) aufweist, mit der das medizinische Fluid (52) aus dem verbundenen oder verbindbaren Behälter (7) in den Schlauch (1), durch die innere Leitung (30) des Schlauchs (1) und durch die mehreren Öffnungen (4) in die Umgebung des Schlauchs (1) zu drücken ist, wobei vorzugsweise die Fördereinrichtung (6) ein Energiespeicherelement, insbesondere zumindest eine gespannte Feder (18), aufweist, wobei die Fördereinrichtung (6) mit Energie aus dem Energiespeicherelement antreibbar ist, wobei besonders bevorzugt mit dem Energiespeicherelement ein Kolben (8) in einem Hohlzylinder als der Behälter (7) in Richtung einer gegenüberliegenden Austragsöffnung anzutreiben ist.

9. Vorrichtung nach einem der vorangehenden Ansprüche, **dadurch gekennzeichnet, dass**
die Vorrichtung einen Konnektor (15) zum Anschließen des Schlauchs (1) an den Behälter (7) für das medizinische Fluid (52) aufweist, wobei der Konnektor (15) einen konischen oder zylindrischen Vorsprung (17) aufweist, der in den Schlauch (1) gesteckt oder geschraubt ist, so dass der Schlauch (1) im Bereich des proximalen Schlauchendes (3) des Schlauchs (1) durch den konischen oder zylindrischen Vorsprung (17) derart gespannt ist, dass der Schlauch (1) am proximalen Schlauchende (3) des Schlauchs (1) fluiddicht mit dem Behälter (7) verbunden ist und fest mit der äußeren Hülse (11) verbunden ist.

10. Vorrichtung nach Anspruch 9, **dadurch gekennzeichnet, dass** der konische oder zylindrische Vorsprung (17) Stege an der Außenseite des konischen oder zylindrischen Vorsprungs (17) aufweist oder der konische oder zylindrische Vorsprung (17) ein Außengewinde (48) aufweist, wobei das Außengewinde (48) oder der konische oder zylindrische Vorsprung (17) einen größeren Außendurchmesser aufweist als der Innendurchmesser des Schlauchs (1).

11. Vorrichtung nach einem der vorangehenden Ansprüche, **dadurch gekennzeichnet, dass**
zumindest am distalen Schlauchende (2) des Schlauchs (1) und/oder im Verschlusselement (5) ein röntgenopakes Material im Schlauch (1) enthalten ist, bevorzugt in einem distalen Teilstück (2) des Schlauchs (1) und, sofern vorhanden, im Verschlusselement (5) ein röntgenopakes Material enthalten ist, besonders bevorzugt auf der gesamten Länge des Schlauchs (1) und, sofern vorhanden, im Verschlusselement (5) enthalten ist, und/oder
zumindest ein Metalldraht (32), zumindest eine Metallspirale und/oder zumindest ein Metallnetz in der inneren Leitung (30) des Schlauchs (1) und/oder in der Schlauchwand des Schlauchs (1) angeordnet ist oder sind, wobei der zumindest eine Metalldraht (32), die zumindest eine Metallspirale und/oder das zumindest ein Metallnetz bevorzugt entlang der gesamten Länge des Schlauchs (1) angeordnet sind.

12. Vorrichtung nach einem der vorangehenden Ansprüche, **dadurch gekennzeichnet, dass**
die Summe der freien Querschnittsflächen aller der mehreren Öffnungen (4) maximal so groß ist wie der freie Querschnitt der inneren Leitung (30) und/oder sich der Schlauch (1) bei einem Innendruck von 500 kPa gegenüber Normaldruck sich radial um maximal 10 Prozent ausdehnt, bevorzugt um maximal 5 Prozent ausdehnt.

13. Verfahren zum Anpassen der Schlauchlänge einer medizinischen Vorrichtung zur lokalen Applikation eines medizinischen Fluids (52), wobei bei dem Verfahren keine medizinische Behandlung eines menschlichen oder tierischen Körpers erfolgt und/oder das medizinische Fluid (52) im Rahmen des Verfahrens nicht an einen menschlichen oder tierischen Körper abgegeben wird, die Vorrichtung aufweisend einen Schlauch (1) mit einer Schlauchwand, wobei der Schlauch (1) mehrere Öffnungen (4) in der Schlauchwand aufweist, wobei die mehreren Öffnungen (4) eine innere Leitung (30) des Schlauchs (1) mit der Umgebung des Schlauchs (1) verbinden, und die Vorrichtung aufweisend eine äußere Hülse (11) zum fluiddichten Verschließen eines proximalen Teils der mehreren Öffnungen (4), wobei die äußere Hülse (11) axial verschiebbar um den Schlauch (1) herum angeordnet ist und wobei die äußere Hülse (11) kürzer ist als der Schlauch (1), so dass die nicht zum proximalen Teil der mehreren Öffnungen (4) gehörenden Öffnungen frei liegen, das Verfahren aufweisend die folgenden Schritte:
A) Verschieben der äußeren Hülse (11) auf dem Schlauch (1) bis eine gewünschte distale Länge des Schlauchs (1) freiliegt;
B) Abschneiden des über die äußere Hülse (11) überstehenden proximalen Teils des Schlauchs (1);
C) Fixieren der äußeren Hülse (11) gegen den Schlauch (1); und
D) Verbinden des neuen proximalen Schlauchendes (3) des Schlauchs (1) mit einem Behälter (7) für das medizinische Fluid (52) oder mit einem Anschluss für einen Behälter (7) für das medizinische Fluid (52), so dass der Behälter (7) flüssigkeitsdurchlässig mit der inneren Leitung (30) des Schlauchs (1) verbunden ist oder verbindbar ist.

14. Verfahren zum Betreiben einer medizinischen Vorrichtung zur lokalen Applikation eines medizinischen Fluids (52) umfassend ein Verfahren nach Anspruch 13, wobei die Schlauchwand des Schlauchs (1) der Vorrichtung eine äußere Wandung (38) aus einem ersten Material aufweist, die radial außen liegend angeordnet ist, und die Schlauchwand eine innere Wandung (40) aus einem zweiten Material aufweist, die radial innen liegend angeordnet ist und die die innere Leitung (30) des Schlauchs (1) begrenzt, **gekennzeichnet durch** die folgenden Schritte:
Schritt E) Einleiten eines medizinischen Fluids (52) in den Schlauch (1);
Schritt F) Ausüben eines Drucks auf das medizinische Fluid (52) in dem Schlauch (1);
Schritt G) Öffnen der mehreren Öffnungen (4) in der inneren Wandung (40) oder in der äußeren Wandung (38) des Schlauchs (1) durch den auf die mehreren Öffnungen (4) wirkenden Druck des medizinischen Fluids (52); und
Schritt H) Austreiben von medizinischem Fluid (52) durch die geöffneten mehreren Öffnungen (4), wobei vorzugsweise ein folgender optionaler Schritt I) erfolgt,
Schritt I) Reduzieren des Drucks auf das medizinische Fluid (52) in dem Schlauch (1) nach Schritt H) und dadurch Schließen der mehreren Öffnungen (4) in der inneren Wandung (40) des Schlauchs (1) oder Verringern des freien Querschnitts der mehreren Öffnungen (4) in der inneren Wandung (40) des Schlauchs (1).

15. Verfahren nach einem der Ansprüche 13 oder 14, **dadurch gekennzeichnet, dass** das Verfahren mit einer Vorrichtung nach einem der Ansprüche 1 bis 12 durchgeführt wird.

## Claims

1. A device for locally applying a medical fluid (52), comprising
a tube (1), the tube (1) being flexibly deformable and comprising a tube wall, wherein the tube (1) comprises a plurality of openings (4) in the tube wall, the plurality of openings (4) connecting an inner line (30) of the tube (1) to the surroundings of the tube (1), and the tube (1) being closed at a distal tube end (2) of the tube (1), wherein a proximal tube end (3) of the tube (1) is liquid-permeably connected or connectable to a container (7) for the medical fluid (52) such that the medical fluid (52) from the container (7) is pushable through the proximal tube end (3) of the tube (1) into the inner line (30) of the tube (1) and can be pushed out through the plurality of openings (4) into the surroundings of the tube (1),
wherein the device comprises an outer sleeve (11) for fluid-tightly closing a subset of the plurality of openings (4), the outer sleeve (11) being axially movably arranged around the tube (1), and the outer sleeve (11) being shorter than the tube (1) such that the distal openings that are not part of the closed subset of the plurality of openings (4) are exposed, wherein
the tube wall comprises an outer wall (38) that is made of a first material and is arranged radially externally, and the tube wall comprises an inner wall (40) that is made of a second material, is arranged radially internally and delimits the inner line (30) of the tube (1), wherein
the outer wall (38) and the inner wall (40) are rigidly interconnected, preferably interconnected over the entire surface.

2. The device according to Claim 1, **characterized in that**
the plurality of openings (4) in the outer wall (38) of the tube wall are open irrespective of the pressure of the medical fluid (52), while the plurality of openings (4) in the inner wall (40) of the tube wall are closed by the medical fluid (52) without pressure being applied and are liquid-permeably openable by means of pressure on the medical fluid (52).

3. The device according to Claim 1 or 2, **characterized in that**
the outer wall (38) of the tube wall absorbs pressure of the medical fluid (52) in the inner line (30), transmitted via the inner wall (40) of the tube wall, without radially expanding by more than 5%, preferably without radially expanding by more than 1%.

4. The device according to any one of the preceding Claims, **characterized in that**
the first material has a larger Shore A hardness than the second material, preferably the first material having a Shore A hardness of more than 60, and the second material having a Shore A hardness of less than 60, and/or
the plurality of openings (4) in the inner wall (40) have an open cross section which, when hydrostatic pressure of 500 kPa is applied by the medical fluid (52), is larger by a factor or two or more than when pressure is not applied, wherein preferably the plurality of openings (4) in the inner wall (40) or the outer wall (38) are slot-shaped.

5. The device according to any one of the preceding, **characterized in that**
the plurality of openings (4) in the tube (1) in the outer wall (38) have a diameter of at most 500 µm, preferably at most 250 µm, and particularly preferably at most 100 µm, and/or
the tube (1) is formed of a coaxial coextrudate, the inner wall (40) consisting of a rubber-elastic polymer, in particular polyurethane or a weakly crosslinked polymer, and the outer wall (38) consisting of a non-rubber-elastic thermoplastic polymer or of a strongly crosslinked polymer, in particular polyamide.

6. The device according to any of the preceding Claims, **characterized in that**
the device comprises a closure element (5), by means of which the tube (1) is fluid-tightly closed at the distal tube end (2) of the tube (1), wherein the inner line (30) of the tube (1) starts at a proximal opening in the proximal tube end (3) of the tube (1) and ends at a distal opening in the distal tube end (2) of the tube (1), the distal opening of the tube (1) preferably being closed by the closure element (5).

7. The device according to any of the preceding Claims, **characterized in that**
the device comprises the container (7) for the medical fluid (52), the container (7) preferably comprising a hollow cylinder having a piston (8) that is axially movable in the hollow cylinder and closes a first end of the hollow cylinder, the hollow cylinder comprising an output opening at an end opposite the first end, said output opening being connected or connectable to the proximal tube end (3) of the tube (1), preferably being connected or being connectable to the proximal tube end (3) of the tube (1) via a manually operable valve element (9) for regulating the flow rate of the medical fluid (52), wherein preferably
a medical fluid (52), in particular a pharmaceutical fluid, is contained in the container (7).

8. The device according to any of the preceding Claims, **characterized in that**
the device comprises a conveying device (6), by means of which the medical fluid (52) is pushable out of the connected or connectable container (7) into the tube (1), through the inner line (30) of the tube (1) and through the plurality of openings (4) into the surroundings of the tube (1), wherein preferably
the conveying device (6) comprises an energy storage element, in particular at least one tensioned spring (18), the conveying device (6) being drivable with energy from the energy storage element, the energy storage element particularly preferably allowing a piston (8) to be driven in a hollow cylinder as the container (7), towards an opposite output opening.

9. The device according to any of the preceding Claims, **characterized in that**
the device comprises a connector (15) for connecting the tube (1) to the container (7) for the medical fluid (52), the connector (15) comprising a conical or cylindrical projection (17) that is inserted or screwed into the tube (1) such that the tube (1) is tensioned by the conical or cylindrical projection (17) in the region of the proximal tube end (3) of the tube (1) such that the tube (1) is fluid-tightly connected to the container (7) at the proximal tube end (3) of the tube (1) and rigidly connected to the outer sleeve (11).

10. The device according to Claim 9, **characterized in that**
the conical or cylindrical projection (17) comprises ribs on the outside of the conical or cylindrical projection (17), or the conical or cylindrical projection (17) comprises an outer thread (48), the outer thread (48) or the conical or cylindrical projection (17) having a larger outer diameter than the inner diameter of the tube (1).

11. The device according to any of the preceding Claims, **characterized in that**
an X-ray-opaque material is contained in the tube (1) at least at the distal tube end (2) of the tube (1) and/or in the closure element (5), an X-ray-opaque material preferably being contained in a distal portion (2) of the tube (1) and, if present, in the closure element (5), particularly preferably being contained over the entire length of the tube (1) and, if present, in the closure element (5), and/or
at least one metal wire (32), at least one metal coil and/or at least one metal mesh is or are arranged in the inner line (30) of the tube (1) and/or in the tube wall of the tube (1), the at least one metal wire (32), the at least one metal coil and/or the at least one metal mesh preferably being arranged along the entire length of the tube (1).

12. The device according to any of the preceding Claims, **characterized in that**
the total of the open cross-sectional areas of all of the plurality of openings (4) is at most as large as the open cross section of the inner line (30), and/or
with an internal pressure of 500 kPa, the tube (1) expands radially by at most 10%, preferably by at most 5%, in relation to normal pressure.

13. A method for adjusting the tube length of a medical device for locally applying a medical fluid (52), wherein in the method, no medical treatment of a human or animal body takes place, and/or the medical fluid (52) is not administered to a human or animal body as part of the method, the device comprising a tube (1) having a tube wall, the tube (1) comprising a plurality of openings (4) in the tube wall, the plurality of openings (4) connecting an inner line (30) of the tube (1) to the surroundings of the tube (1), and the device comprising an outer sleeve (11) for fluid-tightly closing a proximal subset of the plurality of openings (4), the outer sleeve (11) being axially movably arranged around the tube (1), and the outer sleeve (11) being shorter than the tube (1) such that the openings that are not part of the proximal subset of the plurality of openings (4) are exposed, the method comprising the following steps:
A) Moving the outer sleeve (11) on the tube (1) until a desired distal length of the tube (1) is exposed;
B) Cutting the proximal portion of the tube (1) protruding beyond the outer sleeve (11);
C) Fixing the outer sleeve (11) in relation to the tube (1); and
D) Connecting the new proximal tube end (3) of the tube (1) to a container (7) for the medical fluid (52) or to a connection for a container (7) for the medical fluid (52) such that the container (7) is liquid-permeably connected or connectable to the inner line (30) of the tube (1).

14. A method for operating a medical device for locally applying a medical fluid (52), comprising a method according to Claim 13, wherein the tube wall of the tube (1) of the device comprises an outer wall (38) that is made of a first material and is arranged radially externally, and the tube wall comprises an inner wall (40) that is made of a second material, is arranged radially internally and delimits the inner line (30) of the tube (1), **characterized by** the following steps:
Step E) Introducing a medical fluid (52) into the tube (1);
Step F) Exerting pressure on the medical fluid (52) in the tube (1);
Step G) Opening the plurality of openings (4) in the inner wall (40) or in the outer wall (38) of the tube (1) by means of the pressure of the medical fluid (52) acting on the plurality of openings (4);
Step H) Driving out medical fluid (52) through the opened plurality of openings (4);
Step I) Reducing the pressure on the medical fluid (52) in the tube (1) after step H) and thereby closing the plurality of openings (4) in the inner wall (40) of the tube (1) or decreasing the open cross section of the plurality of openings (4) in the inner wall (40) of the tube (1).

15. The method according to any one of Claims 13 or 14, **characterized in that** the method is carried out using a device according to any of Claims 1 to 12.

## Revendications

1. Dispositif d'application locale d'un fluide médical (52) présentant un tuyau (1), lequel est déformable de manière flexible et lequel présente une paroi de tuyau,
le tuyau (1) présentant plusieurs ouvertures (4) dans la paroi de tuyau, les plusieurs ouvertures (4) reliant une conduite intérieure (30) du tuyau (1) avec l'environnement du tuyau (1) et le tuyau (1) étant fermé à une extrémité de tuyau distale (2) du tuyau (1),
une extrémité de tuyau proximale (3) du tuyau (1) étant ou pouvant être reliée de manière perméable aux liquides avec un récipient (7) pour le fluide médical (52) de telle façon que le fluide médical (52) peut être pressé hors du récipient (7) à travers l'extrémité de tuyau proximale (3) du tuyau (1) dans la conduite intérieure (30) du tuyau (1) et peut être extrait dans l'environnement du tuyau (1) à travers les plusieurs ouvertures (4),
le dispositif présentant une douille extérieure (11) pour la fermeture étanche aux fluides d'une partie des plusieurs ouvertures (4), la douille extérieure (11) étant disposée de manière déplaçable axialement autour du tuyau (1) et la douille extérieure (11) étant plus courte que le tuyau (1), de telle sorte que les ouvertures distales n'appartenant pas à la partie fermée des plusieurs ouvertures (4) soient exposées,
la paroi de tuyau présentant une paroi extérieure (38) en un premier matériau, laquelle est disposée radialement à l'extérieur et la paroi de tuyau présentant une paroi intérieure (40) en un deuxième matériau, laquelle est disposée radialement à l'intérieur et laquelle limite la conduite intérieure (30) du tuyau (1),
la paroi extérieure (38) et la paroi intérieure (40) étant reliées solidement l'une à l'autre, de préférence reliées l'une à l'autre sur toute leur surface.

2. Dispositif selon la revendication 1, **caractérisé en ce que**
les plusieurs ouvertures (4) dans la paroi extérieure (38) de la paroi de tuyau sont ouvertes indépendamment de la pression du fluide médical (52), tandis que les plusieurs ouvertures (4) dans la paroi intérieure (40) de la paroi de tuyau sont fermées sans sollicitation par pression par le fluide médical (52) et peuvent être ouvertes de manière perméable aux liquides par une pression sur le fluide médical (52).

3. Dispositif selon la revendication 1 ou 2, **caractérisé en ce que**
la paroi extérieure (38) de la paroi de tuyau absorbe une pression du fluide médical (52) dans la conduite intérieure (30) transmise à travers la paroi intérieure (40) de la paroi de tuyau, sans se dilater radialement de plus de 5 %, de préférence sans se dilater radialement de plus de 1 %.

4. Dispositif selon l'une quelconque des revendications précédentes, **caractérisé en ce que** le premier matériau a une dureté Shore-A supérieure à celle du deuxième matériau, le premier matériau ayant de préférence une dureté Shore-A supérieure à 60 et le deuxième matériau ayant une dureté Shore-A inférieure à 60 et/ou les plusieurs ouvertures (4) dans la paroi intérieure (40) présentent, lors d'une sollicitation par pression avec une pression hydrostatique de 500 kPa par le fluide médical (52), une section transversale libre plus grande d'un facteur de deux ou supérieur à celle sans sollicitation par pression,
les plusieurs ouvertures (4) dans la paroi intérieure (40) ou dans la paroi extérieure (38) étant de préférence en forme de fentes.

5. Dispositif selon l'une quelconque des revendications précédentes, **caractérisé en ce que** les plusieurs ouvertures (4) dans le tuyau (1) dans la paroi extérieure (38) ont un diamètre de maximum 500 µm, de préférence de maximum 250 µm et de manière particulièrement préférée de maximum 100 µm et/ou
le tuyau (1) est formé d'un coextrudat coaxial, la paroi intérieure (40) étant composée d'un polymère caoutchouteux, en particulier de polyuréthane, ou d'un polymère faiblement réticulé et la paroi extérieure (38) étant composée d'un polymère thermoplastique non caoutchouteux ou d'un polymère fortement réticulé, en particulier de polyamide.

6. Dispositif selon l'une quelconque des revendications précédentes, **caractérisé en ce que** le dispositif présente un élément de fermeture (5), au moyen duquel le tuyau (1) est fermé de manière étanche aux liquides à l'extrémité de tuyau distale (2) du tuyau (1), la conduite intérieure (30) du tuyau (1) commençant à une ouverture proximale dans l'extrémité de tuyau proximale (3) du tuyau (1) et terminant à une ouverture distale dans l'extrémité de tuyau distale (2) du tuyau (1), l'ouverture distale du tuyau (1) étant de préférence fermée par l'élément de fermeture (5).

7. Dispositif selon l'une quelconque des revendications précédentes, **caractérisé en ce que** le dispositif présente le récipient (7) pour le fluide médical (52), le récipient (7) présentant de préférence un cylindre creux comprenant un piston (8) pouvant être déplacé axialement dans le cylindre creux, lequel ferme une première extrémité du cylindre creux, le cylindre creux présentant une ouverture de décharge à une extrémité opposée à la première extrémité, laquelle est ou peut être reliée avec l'extrémité de tuyau proximale (3) du tuyau (1), est ou peut être de préférence reliée à l'extrémité de tuyau proximale (3) du tuyau (1) à travers un élément de soupape (9) actionnable manuellement pour la régulation de la vitesse d'écoulement du fluide médical (52),
un fluide médical (52), en particulier un fluide pharmaceutique, étant de préférence contenu dans le récipient (7).

8. Dispositif selon l'une quelconque des revendications précédentes, **caractérisé en ce que** le dispositif présente un système de transport (6), au moyen duquel le fluide médical (52) peut être pressé hors du récipient (7) relié ou pouvant être relié dans le tuyau (1), à travers la conduite intérieure (30) du tuyau (1) et à travers les plusieurs ouvertures (4) dans l'environnement du tuyau (1),
le système de transport (6) présentant de préférence un élément de stockage d'énergie, en particulier au moins un ressort (18) tendu, le système de transport (6) pouvant être entraîné au moyen d'énergie de l'élément de stockage d'énergie, un piston (8) dans un cylindre creux en tant que le récipient (7) pouvant entre entraîné de manière particulièrement préférée au moyen de l'élément de stockage d'énergie dans la direction d'une ouverture de décharge opposée.

9. Dispositif selon l'une quelconque des revendications précédentes, **caractérisé en ce que** le dispositif présente un connecteur (15) pour le raccord du tuyau (1) au récipient (7) pour le fluide médical (52), le connecteur (15) présentant une saillie (17) conique ou cylindrique, laquelle est enfichée ou vissée dans le tuyau (1), de sorte que le tuyau (1) soit tendu, dans la zone de l'extrémité de tuyau proximale (3) du tuyau (1), par la saillie (17) conique ou cylindrique de telle façon que le tuyau (1) soit relié à l'extrémité de tuyau proximale (3) du tuyau (1) de manière étanche aux fluides avec le récipient (7) et soit relié solidement avec la douille extérieure (11).

10. Dispositif selon la revendication 9, **caractérisé en ce que**
la saillie (17) conique ou cylindrique présente des traverses sur le côté extérieur de la saillie (17) conique ou cylindrique ou la saillie (17) conique ou cylindrique présente un filetage extérieur (48), le filetage extérieur (48) ou la saillie (17) conique ou cylindrique présentant un diamètre extérieur plus grand que le diamètre intérieur du tuyau (1).

11. Dispositif selon l'une quelconque des revendications précédentes, **caractérisé en ce que** un matériau radio-opaque est contenu dans le tuyau (1) au moins à l'extrémité de tuyau distale (2) du tuyau (1) et/ou dans l'élément de fermeture (5), un matériau radio-opaque est contenu de préférence dans une partie distale (2) du tuyau (1) et, s'il existe, dans l'élément de fermeture (5), contenu de manière particulièrement préférée sur la longueur totale du tuyau (1) et, s'il existe, dans l'élément de fermeture (5) et/ou au moins un fil métallique (32), au moins une spirale métallique et/ou au moins un filet métallique est ou sont disposé(s) dans la conduite intérieure (30) du tuyau (1) et/ou dans la paroi de tuyau du tuyau (1), l'au moins un fil métallique (32), l'au moins une spirale métallique et/ou l'au moins un filet métallique étant disposés de préférence le long de la longueur totale du tuyau (1).

12. Dispositif selon l'une quelconque des revendications précédentes, **caractérisé en ce que** la somme des sections transversales libres de toutes les plusieurs ouvertures (4) est au maximum aussi grande que la section transversale libre de la conduite intérieure (30) et/ou le tuyau (1), pour une pression intérieure de 500 kPa par rapport à la pression normale, se dilate radialement de maximum 10 pour cent, de préférence de maximum 5 pour cent.

13. Procédé d'adaptation de la longueur de tuyau d'un dispositif médical pour l'application locale d'un fluide médical (52), le procédé ne réalisant aucun traitement médical d'un corps humain ou animal et/ou le fluide médical (52) n'étant pas transmis, dans le cadre du procédé, à un corps humain ou animal, le dispositif présentant un tuyau (1) comprenant une paroi de tuyau, le tuyau (1) présentant plusieurs ouvertures (4) dans la paroi de tuyau, les plusieurs ouvertures (4) reliant une conduite intérieure (30) du tuyau (1) avec l'environnement du tuyau (1) et le dispositif présentant une douille extérieure (11) pour la fermeture étanche aux fluides d'une partie proximale des plusieurs ouvertures (4), la douille extérieure (11) étant disposée de manière déplaçable axialement autour du tuyau (1) et la douille extérieure (11) étant plus courte que le tuyau (1), de telle sorte que les ouvertures n'appartenant pas à la partie proximale des plusieurs ouvertures (4) soient exposées, le procédé présentant les étapes suivantes :
A) déplacement de la douille extérieure (11) sur le tuyau (1) jusqu'à ce qu'une longueur distale souhaitée du tuyau (1) soit exposée ;
B) découpe de la partie proximale du tuyau (1) faisant saillie au-delà de la douille extérieure (11) ;
C) fixation de la douille extérieure (11) contre le tuyau (1) ; et
D) liaison de la nouvelle extrémité de tuyau proximale (3) du tuyau (1) à un récipient (7) pour le fluide médical (52) ou avec un raccord pour un récipient (7) pour le fluide médical (52), de sorte que le récipient (7) soit ou puisse être relié de manière perméable aux liquides avec la conduite intérieure (30) du tuyau (1).

14. Procédé pour faire fonctionner un dispositif médical d'application locale d'un fluide médical (52) comprenant un procédé selon la revendication 13, la paroi de tuyau du tuyau (1) du dispositif présentant une paroi extérieure (38) en un premier matériau, laquelle est disposée radialement à l'extérieur et la paroi de tuyau présentant une paroi intérieure (40) en un deuxième matériau, laquelle est disposée radialement à l'intérieur et laquelle limite la conduite intérieure (30) du tuyau (1), **caractérisé par** les étapes suivantes :
étape E) introduction d'un fluide médical (52) dans le tuyau (1) ;
étape F) application d'une pression sur le fluide médical (52) dans le tuyau (1) ;
étape G) ouverture des plusieurs ouvertures (4) dans la paroi intérieure (40) ou dans la paroi extérieure (38) du tuyau (1) au moyen de la pression du fluide médical (52) agissant sur les plusieurs ouvertures (4) ; et
étape H) passage de fluide médical (52) à travers les plusieurs ouvertures (4) ouvertes, une étape I) optionnelle suivante étant de préférence effectuée,
étape I) réduction de la pression sur le fluide médical (52) dans le tuyau (1) après l'étape H) et ainsi fermeture des plusieurs ouvertures (4) dans la paroi intérieure (40) du tuyau (1) ou réduction de la section transversale libre des plusieurs ouvertures (4) dans la paroi intérieure (40) du tuyau (1).

15. Procédé selon l'une quelconque des revendications 13 ou 14, **caractérisé en ce que** le procédé est exécuté avec un dispositif selon l'une quelconque des revendications 1 à 12.
